(19) Europäisches Patentamt
European Patent Office
Office européen des brevets

(11) EP 2 264 182 A1

(12) EUROPEAN PATENT APPLICATION
published in accordance with Art. 153(4) EPC

(43) Date of publication:
22.12.2010 Bulletin 2010/51

(21) Application number: 09714119.6

(22) Date of filing: 27.02.2009

(51) Int Cl.:
*C12Q 1/68* (2006.01)
*C12N 15/09* (2006.01)
*C12Q 1/04* (2006.01)
*G01N 33/48* (2006.01)
*G01N 33/53* (2006.01)

(86) International application number:
PCT/JP2009/053700

(87) International publication number:
WO 2009/107785 (03.09.2009 Gazette 2009/36)

(84) Designated Contracting States:
AT BE BG CH CY CZ DE DK EE ES FI FR GB GR HR HU IE IS IT LI LT LU LV MC MK MT NL NO PL PT RO SE SI SK TR
Designated Extension States:
AL BA RS

(30) Priority: 28.02.2008 JP 2008048197

(71) Applicant: Sysmex Corporation
Kobe-shi, Hyogo 651-0073 (JP)

(72) Inventors:
- IKEDA, Masafumi
Kobe-shi
Hyogo 651-0073 (JP)
- UGA, Hitoshi
Kobe-shi
Hyogo 651-0073 (JP)
- TANAKA, Satoshi
Kobe-shi
Hyogo 651-0073 (JP)
- MIYAMOTO, Yoshiaki
Kobe-shi
Hyogo 651-0073 (JP)
- YANAGIDA, Masatoshi
Kobe-shi
Hyogo 651-0073 (JP)
- KURATA, Hirokazu
Kobe-shi
Hyogo 651-0073 (JP)
- KADOWAKI, Masakazu
Kobe-shi
Hyogo 651-0073 (JP)

(74) Representative: Paemen, Liesbet R.J. et al
De Clercq & Partners cvba
Edgard Gevaertdreef 10 a
9830 Sint-Martens-Latem (BE)

# (54) MARKER FOR DETECTION OF IL-17-PRODUCING HELPER T-CELL, AND METHOD FOR DETECTION OF IL-17-PRODUCING HELPER T-CELL

(57) Disclosed is a polynucleotide marker or a protein marker for use in the specific detection of an IL-17-producing helper T-cell (a Th17 cell). Also disclosed is a method for detecting a Th17 cell, which is **characterized by** detecting the occurrence of the polynucleotide marker or the protein marker.

EP 2 264 182 A1

## Description

### TECHNICAL FIELD

The present invention relates to a marker for detecting IL-17-producing helper T-cells (hereinafter referred to as "Th17 cells") and a method for detecting Th17 cells.

### BACKGROUND ART

Rheumatoid arthritis (hereinafter referred to as "RA") is the systemic inflammatory autoimmune disease whose main clinical symptom is arthritis. The state of RA is diagnosed by rational symptoms such as joint pain or by visual procedures such as the observations on the extent of swelling or bone X-ray. However, no quantitative index has been established. Thus, no quantitative method for continuously monitoring the therapeutic effects has been established under the current state of the art.

RA is the autoimmune disease, and its pathogenesis has not been elucidated. It is considered that bacterial infections and the like trigger an inflammation in joint tissues via complicated networks of immunocytes and cytokines. Helper T-cells are responsible for immune reactions. Immature helper T-cells (naïve T-cells) are differentiated into helper T-cells when an antigen is presented by antigen-presenting cells. When specific cytokines are present at this time, naïve T-cells are differentiated into four types of the cells, which are helper T-cells producing interferon (IFN)-γ (Th1 cells), helper T-cells producing interleukin (IL)-4 (Th2 cells), helper T-cells producing IL-17 cells (Th 17 cells) and regulatory T-cells having immunosuppressive effects (Treg cells).

It has been shown that among these helper T-cells, Th 17 cells can be involved in the onset of RA.

It has been suggested that IL-17 is deeply involved in the formation of pathological condition and in particular joint and bone deformities because the level of IL-17 is significantly higher in synovial fluid of RA patients than in that of the patients of osteoarthritis and T-cells in synovial tissue from RA patients include IL-17 positive cells (see Japanese Unexamined Patent Publication No. 2000-186046; Patent Document 1). Patent Document 1 discloses that IL-17 can be used as a diagnostic marker of RA.

Japanese Unexamined Patent Publication No. 2007-506100 (Patent Document 2) discloses that the analysis of cytokines in peripheral blood serum of RA patients revealed that the levels of IFN-γ, IL-1β, TNF-α, G-CSF, GM-CSF, IL-6, IL-4, IL-10, IL-13, IL-5 and IL-7 were significantly high and the levels of IL-2, CXCLB/IL-8, IL-12 and CCL2/MCP-1 were not high in RA patients.

According to the studies by Ivanov et al. (Cell, 2006, 126, p.1121-1133; Non-patent Document 1), Stumhofer et al. (Nature Immunology, 2006, vol.7, p.937-945; Non-patent Document 2), and Wilson et al. (Nature Immunology, 2007, vol.8, p.950-957; Non-patent Document 3), the following facts have been shown about Th17 cells:

- a nuclear receptor called RORγt has an important role in the differentiation of Th17 cells;
- IL-6, IL-23 and TGF-β induce the differentiation of immature helper T-cells (naïve T-cells) to Th17 cells;
- they express IL-17A, IL-17F, IL-6, IL-22, IL-26, TNF, IFN-γ and CCL20; and
- IL-23 receptor and IL-12 receptor β are located on the surface of Th17 cells.

In the above Non-patent Documents 1 to 3, the amount of IL-17 is measured by enzyme linked immunosorbent assay (ELISA) using antibodies specific to IL-17.

The relations between Th17 cells and autoimmune diseases, preferably RA, ulcerative colitis, Crohn's disease, multiple sclerosis (encephalitis and/or myelitis), particularly preferably RA and multiple sclerosis (encephalitis) may be more deeply understood by establishing a method which is able to not only measure the amount of IL-17 but also detect Th17 cells per se.

Patent Document 1: Japanese Unexamined Patent Publication No. 2000-186046
Patent Document 2: Japanese Unexamined Patent Publication No. 2007-506100
Non-patent Document 1: Ivanov et al., "The Orphan Nuclear Receptor RORγt Directs the Differentiation Program of Proinflammatory IL-17+ T Helper Cells", Cell, 2006, 126, p.1121-1133
Non-patent Document 2: Stumhofer et al., "Interleukin 27 negatively regulates the development of interleukin 17-producing T helper cells during chronic inflammation of the central nervous system" Nature Immunology, 2006, vol. 7, p.937-945
Non-patent Document 3: Wilson et al., "Development, cytokine profile and function of human interleukin 17-producing helper T cells" Nature Immunology, 2007, vol.8, p.950-957

## SUMMARY OF INVENTION

Technical Problem

The inventors aimed to find molecular markers that make it possible to specifically detect Th17 cells, particularly molecular markers that are highly expressed in the diseases in which Th17 cells are considered to be involved.

Means for Solving the Problems

First, the inventors identified genes and expressed sequence tags (ESTs) which are specifically expressed in Th17 cells differentiated from naïve T cells isolated from the spleen of mice. The inventors then extracted the genes and ESTs which are highly expressed in model mice of the diseases in which Th17 cells are considered to be involved (arthritis models and/or encephalomyelitis models) from the genes and ESTs identified in Th17 cells and completed the present invention.

Thus, the present invention provides a polynucleotide marker for detecting Th 17 cells which is a polynucleotide selected from the group consisting of:

a gene encoding a cytokine selected from the group consisting of Interleukin 17A; Interleukin 22; and Interleukin tifb;
a gene encoding a chemokine which is Chemokine, CC motif, ligand 20;
a gene encoding a membrane protein selected from the group consisting of Interleukin 17 receptor E; Interleukin 1 receptor 1; Interleukin 27receptor A; G protein-coupled receptor 15; Stabilin 1; Podoplanin; Transmembrane and immunoglobulin domain containing 1; Melanocortin 2 receptor; Transmembrane protein 176A; Progestin and adipoQ receptor family member VIII; Claudin domain containing 1; ELOVL family member 7; Lymphocyte antigen 6 complex, locus K; G protein-coupled receptor 183 (Epstein-Barr virus induced gene 2); Killer cell lectin-like receptor subfamily B member 1F; Transferrin receptor 2; Neuron specific gene family member 2; Transmembrane protein 176B; Amyloid beta (A4) precursor-like protein 2; Immunoglobulin joining chain; Adhesion molecule with Ig like domain 2; Fc receptor, IgG, low affinity IIb; Cannabinoid receptor 2; Tumor necrosis factor receptor superfamily, member 14; Aquaporin 3; C1q and tumor necrosis factor related protein 3; Synaptotagmin XI; Potassium channel tetramerisation domain containing 12; Apolipoprotein L 7b (expressed sequence BC085284); Apolipoprotein L7e (similar to apolipoprotein L, 3); Solute carrier family 34 (member 3); Retinol binding protein 1, cellular; similar to cellular retinol binding protein I; Potassium large conductance calcium-activated channel (subfamily M, beta member 4); similar to calcium activated potassium channel beta 4 subunit; SYS 1 Golgi-localized integral membrane protein homolog (RIKEN cDNA 2610042014 gene); and Solute carrier family 38, member 6 (expressed sequence AW322671);

a gene encoding a transcription/translation factor selected from the group consisting of POU domain, class 2, associating factor 1; Transcription factor 7 (T-cell specific); WW domain containing transcription regulator 1; Trichorhinophalangeal syndrome I; Centrosomal protein 290; and Ataxin 2 binding protein 1;
a gene encoding a signaling molecule selected from the group consisting of Ras-related associated with diabetes; Breast cancer anti-estrogen resistance 3; Rab38 (member of RAS oncogene family); Centaurin, gamma 2; SH3 and PX domains 2B; FERM, RhoGEF and pleckstrin domain protein 2; Disabled homolog 2; B-cell leukemia/lymphoma 2 related protein A1a; B-cell leukemia/ lymphoma 2 related protein A1b; and B-cell leukemia/ lymphoma 2 related protein A1d;
a gene encoding an adhesion molecule which is Transforming growth factor beta induced;

a gene encoding an enzyme selected from the group consisting of Cytochrome P450, family 1, subfamily b, polypeptide 1; EH-domain containing 3; Matrix metallopeptidase 13; Carboxypeptidase D; Carbonic anhydrase 13; Glucosaminyl (N-acetyl) transferase 2, 1-branching enzyme; UDP glucuronosyltransferase 1 family, polypeptide A2; UDP glucuronosyltransferase 1 family, polypeptide A6A; UDP glucuronosyltransferase 1 family, polypeptide A6B; UDP glucuronosyltransferase 1 family, polypeptide A10; UDP glucuronosyltransferase 1 family, polypeptide A7C; UDP glucuronosyltransferase 1 family, polypeptide A5; UDP glucuronosyltransferase 1 family, polypeptide A9; UDP glucuronosyltransferase 1 family, polypeptide A1; Similar to UDP glycosyltransferase 1 family, polypeptide A8; UDP-GlcNAc: betaGal beta-1,3-N-acetylglucosaminyltransferase 8; Bone morphogenetic protein 1; Uridine phosphorylase 1; Myosin III B; beta-site APP-cleaving enzyme 2; Mast cell protease 1; COX10 homolog, cytochrome c oxidase assembly protein, heme A: farnesyltransferase; Dynamin 3; Acid phosphatase, prostate; Phosphodiesterase 5A (cGMP-specific); Patatin-like phospholipase domain containing 7; RIKEN cDNA 1300007F04 gene; RIKEN cDNA 1810062018 gene; Phosphatase, orphan 1 (expressed sequence AI447357, ABI gene family, member 3); and Exostoses (multiple) 1;

a gene encoding an enzyme inhibitor selected from the group consisting of Serine (or cysteine) peptidase inhibitor, clade B, member 1a; Protein phosphatase 1, regulatory (inhibitor) subunit 14c; Protein kinase inhibitor beta (cAMP dependent, testis specific); Tissue inhibitor of metalloproteinase 1; Serine (or cysteine) peptidase inhibitor, clade I, member 1; Amyloid beta (A4) precursor protein; and WAP four-disulfide core domain 2;

a gene encoding a secretory protein which is Cysteine-rich secretory protein LCCL domain containing 2;

a gene encoding a structural protein selected from the group consisting of Plastin 1 (expressed sequence AI427122); immunoglobulin heavy chain complex; immunoglobulin heavy chain 1a (serum IgG2a); immunoglobulin heavy chain 2 (serum IgA); immunoglobulin heavy chain Ia; immunoglobulin heavy chain (J558 family); immunoglobulin heavy chain (gamma polypeptide); similar to immunoglobulin mu-chain; similar to immunoglobulin heavy chain V region 3 precursor; immunoglobulin heavy chain variable region; similar to immunoglobulin heavy chain V region 102 precursor; immunoglobulin heavy chain 3; Nebulette; Lumican; Bactericidal/permeability-increasing protein-like 2; Kelch-like 8; Tripartite motif protein 2; PDZ and LIM domain 5; Keratin 86; Kinesin family member 3C; Kinesin family member 1B; and Kinesin family member 5C;

a gene selected from Sex comb on midleg-like 4; High mobility group AT-hook 2, pseudogene 1; RIKEN cDNA 2310007L24 gene; RIKEN cDNA 2310002J15 gene; Family with sequence similarity 101, member B (RIKEN cDNA 1500005K14 gene); expressed sequence AI646023; and GRAM domain containing 3; and

an expressed sequence tag (EST) selected from TOX high mobility group box family member 2 (expressed sequence AI851523); RIKEN cDNA 6030439D06 gene; RIKEN cDNA 9030418K01 gene; expressed sequence AU015680; a polynucleotide having the sequence of SEQ ID NO: 1 ; and a polynucleotide having the sequence of SEQ ID NO: 2.

The present invention preferably relates to a polynucleotide marker for detecting Th 17 cells which is a polynucleotide selected from the group consisting of:

a gene encoding a cytokine selected from the group consisting of Interleukin 17A; Interleukin 22; and Interleukin tifb;
a gene encoding a membrane protein selected from the group consisting of Interleukin 1 receptor 1; Interleukin 27receptor A; G protein-coupled receptor 15; Stabilin 1; Apolipoprotein L 7b (expressed sequence BC085284); Apolipoprotein L7e (similar to apolipoprotein L, 3); C1q and tumor necrosis factor related protein 3; Cannabinoid receptor 2; Fc receptor, IgG, low affinity IIb; G protein-coupled receptor 183 (Epstein-Barr virus induced gene 2); Retinol binding protein 1, cellular; similar to cellular retinol binding protein I; Lymphocyte antigen 6 complex, locus K; Solute carrier family 38, member 6 (expressed sequence AW322671); Synaptotagmin XI; Transmembrane protein 176A; Transmembrane protein 176B; and Tumor necrosis factor receptor superfamily, member 14;

a gene encoding a transcription/translation factor selected from Transcription factor 7 (T-cell specific); and WW domain containing transcription regulator 1

a gene encoding a signaling molecule selected from the group consisting of B-cell leukemia/lymphoma 2 related protein A1a; B-cell leukemia/lymphoma 2 related protein A1b; B-cell leukemia/ lymphoma 2 related protein A1d; Disabled homolog 2; Ras-related associated with diabetes; and SH3 and PX domains 2B;

a gene encoding an adhesion molecule which is Transforming growth factor beta induced;

a gene encoding an enzyme selected from the group consisting of Acid phosphatase, prostate; UDP-GlcNAc: betaGal beta-1,3-N-acetylglucosaminyltransferase 8; Bone morphogenetic protein 1; Carbonic anhydrase 13; Cytochrome P450, family 1, subfamily b, polypeptide 1; Glucosaminyl (N-acetyl) transferase 2, 1-branching enzyme; UDP glucuronosyltransferase 1 family, polypeptide A2; UDP glucuronosyltransferase 1 family, polypeptide A6A; UDP glucuronosyltransferase 1 family, polypeptide A6B; UDP glucuronosyltransferase 1 family, polypeptide A10; UDP glucuronosyltransferase 1 family, polypeptide A7C; UDP glucuronosyltransferase 1 family, polypeptide A5; UDP glucuronosyltransferase 1 family, polypeptide A9; UDP glucuronosyltransferase 1 family, polypeptide A1; Similar to UDP glycosyltransferase 1 family, polypeptide A8; Matrix metallopeptidase 13; Phosphodiesterase 5A (cGMP-specific); Phosphatase, orphan 1 (expressed sequence AI447357, ABI gene family, member 3); and Uridine phosphorylase 1;

a gene encoding an enzyme inhibitor selected from Serine (or cysteine) peptidase inhibitor, clade B, member 1a; and Tissue inhibitor of metalloproteinase 1;

a gene encoding a secretory protein which is Cysteine-rich secretory protein LCCL domain containing 2;

a gene encoding a structural protein selected from Kinesin family member 5C; and Lumican; and

an expressed sequence tag (EST) selected from RIKEN cDNA 6030439D06 gene; and RIKEN cDNA 9030418K01 gene.

The present invention is more preferably a polynucleotide marker for detecting Th 17 cells which is a polynucleotide selected from the group consisting of:

a gene encoding a cytokine which is Interleukin 17A;
a gene encoding a membrane protein selected from the group consisting of Interleukin 1 receptor 1; Apolipoprotein L 7b (expressed sequence BC085284); Apolipoprotein L7e (similar to apolipoprotein L, 3); Cannabinoid receptor 2; Fc receptor, IgG, low affinity IIb; Solute carrier family 38, member 6 (expressed sequence AW322671); and Transmembrane protein 176A;
a gene encoding a signaling molecule selected from the group consisting of B-cell leukemia/lymphoma 2 related protein A1a; B-cell leukemia/lymphoma 2 related protein A1b; and B-cell leukemia/ lymphoma 2 related protein A1d;
a gene encoding an enzyme which is Matrix metallopeptidase 13;

a gene encoding an enzyme inhibitor which is Tissue inhibitor of metalloproteinase 1; and
a gene encoding a secretory protein which is Cysteine-rich secretory protein LCCL domain containing 2.

The present invention also provides a protein marker for detecting Th17 cells consisting of a protein encoded by the above gene.
The present invention further provides a method for detecting Th17 cells comprising detecting the presence of the polynucleotide marker for detecting Th 17 cells or the protein marker for detecting Th 17 cells in a sample containing cells. In addition, the present invention provides a DNA chip or microarray and a probe including a primer for detecting the polynucleotide marker for detecting Th17 cells, an antibody for detecting the protein marker for detecting Th17 cells, and a kit for detecting Th17 cells comprising at least one of the above.

Effect of the Invention

Th17 cells can be specifically detected by detecting the present polynucleotide or protein marker. Accordingly, Th17 cells can be isolated from samples containing various cells by using the present marker. For example, Th17 cells can be specifically detected in samples containing cells such as tissues obtained from patients by using the present marker. Therefore, it is considered that the morbidity of the patients suffering from diseases in which Th17 cells are considered to be involved such as autoimmune diseases, preferably RA, ulcerative colitis, Crohn's disease and multiple sclerosis (encephalitis and/or myelitis), particularly preferably RA and multiple sclerosis (encephalitis), can be diagnosed.

## BEST MODE FOR CARRYING OUT THE INVENTION

The polynucleotide marker for detecting Th17 cells of the present invention is selected from the polynucleotide selected from the group consisting of the above genes and ESTs, and variants and fragments thereof.
The polynucleotide marker is the polynucleotide, variant or fragment thereof which has been found to be specifically present in Th17 cells rather than in other helper T-cells differentiated from naïve T-cells (Th1, Th2 and Treg cells). The polynucleotide marker is preferably the polynucleotide, variant or fragment thereof which has been found to be highly expressed in the above described autoimmune disease mouse models. The polynucleotide marker is more preferably the polynucleotide, variant or fragment thereof which has been found to be correlated with IL-17 expression level or to the pathological conditions.
Therefore, by detecting the polynucleotide marker, Th17 cells can be distinguished from Th1, Th2 and Treg cells and can be specifically identified, and this can be used as a measure for diseases in which Th17 cells are considered to be involvedin vivo.
As used herein, the term "gene" has the same meaning as that is commonly recognized in the art, and refers to a part of a genome which is transcribed in mRNA and translated into a protein.
As used herein, the term "expressed sequence tag (EST)" has the same meaning as that is commonly recognized in the art, and refers to a partial sequence of a gene which serves as a mark for the fact that the gene is trancribed into mRNA.
The term "signaling molecule" means a series of signaling transducers which locate in cell membranes, cytoplasms, nuclei and the like and are activated in response to the intracellular and extracellular stimuli. The term "adhesion molecule" means a group of molecules which locate on the surface of cell membranes and bind to extracellular matrices or other cell surface molecules to elicit a physical adhesion, signal transduction, molecular structural change or the like. The term "structural protein" means a group of molecules which locate predominantly in the cells and are responsible for construction and maintenance of cell morphology, movement, translocation of signaling molecules or the like.
As used herein, the expression that a polynucleotide is "specifically expressed" in Th17 cells means that the expression level of the polynucleotide in Th 17 cells is significantly higher than the expression level of the polynucleotide in cells other than Th17 cells. Specifically, it means that the expression level of the polynucleotide in Th17 cells is about three times or more the expression level of the polynucleotide in cells other than Th17 cells. Preferably, the expression level of the polynucleotide in Th17 cells is about three times or more the expression level of the polynucleotide in helper T-cells other than Th17 cells (Th1, Th2 and Treg cells).
The nucleotide sequences of the present polynucleotide markers are already known. They can be obtained from, for example, Unigene (a database provided by National Center for Biotechnology Information (NCBI) of National Library of Medicine). Unigene codes for the sequences of the present polynucleotide markers are specified in Table 2 below.
As used herein, "variant" of a polynucleotide means a polynucleotide in which a mutation has been introduced that does not alter the nature of the protein encoded by the above described genes or a gene detected by the above described ESTs. Such mutation includes a deletion, substitution or addition of one or more nucleotides to the known nucleic acid sequence of the above described gene or EST.

The variant has generally at least 80%, more preferably at least 85%, further preferably at least about 90% and particularly preferably at least 95% homology with the known nucleic acid sequence of the above described gene or EST.

As used herein, the homology of nucleic acid and amino acid sequences means the one calculated in BLASTN, BLASTP, BLASTX or TBLASTN (e.g. available from http://www.ncbi.nlm.nih.gov) with default settings.

The polynucleotide marker may be any of DNA or RNA, and may be the gene per se (DNA), mRNA, cDNA or cRNA.

Th17 cells can also be detected by detecting the protein encoded by the gene which is the polynucleotide marker of the present invention. Thus, the present invention also provides the protein marker for detecting Th17 cells consisting of the protein encoded by the above described gene.

The sequence of such protein marker can be obtained based on the nucleic acid sequence of the polynucleotide marker obtained from Unigene and the like. It can also be obtained from databases provided by NCBI and the like. NCBI code numbers for the amino acid sequences of the present protein markers are specified in Table 2 below.

The protein marker for detecting Th17 cells may be selected from proteins encoded by the above described genes, functionally equivalent variants thereof and fragments thereof.

"Functionally equivalent variant" of the protein means a protein into which a mutation has been introduced that does not alter functions of the protein. Such mutation includes a deletion, substitution or addition of one or more amino acids to the known amino acid sequence of the above protein.

The functionally equivalent variant of the protein has generally at least 80%, more preferably at least 85%, further preferably at least about 90% and particularly preferably at least 95% homology with the known amino acid sequence of the protein.

A molecule that can specifically hybridize to the polynucleotide marker can be used for the detection of the marker, making it useful as a probe for detecting Th17 cells. The probe may be a nucleic acid probe such as DNA or RNA, or a peptide probe that can specifically hybridize to the polynucleotide marker. The probe for detecting Th17 cells is preferably a nucleic acid probe, particularly a DNA probe for detecting the polynucleotide marker.

As used herein, the expression "can specifically hybridize" means that it can hybridize to a target nucleic acid molecule (the polynucleotide marker) under stringent conditions.

As used herein, "stringent conditions" means conditions under which the probe for detecting Th 17 cells can hybridize to the target polynucleotide marker with a detectably higher extent than it does to a polynucleotide other than the target polynucleotide marker (e.g. more than at least two times the background). The stringent conditions generally depend on the sequences and varies depending on the conditions. Generally, the stringent conditions are selected such that the hybridization temperature is about 5˚C lower than a thermal melting point of the specific sequence under a certain ionic strength and pH. This Tm is a temperature at which 50% of the complementary probe hybridizes to the target sequence in equilibrium (under a certain ionic strength, pH and nucleic acid composition).

Such conditions may be the ones which are used in common hybridization techniques between polynucleotides such as PCR, microarray or Southern blotting. Specifically, it may include conditions of pH 7.0 to 9.0, a salt concentration of lower than about 1.5M Na-ion, more specifically about 0.01 to 1.0 M Na-ion concentration (or other salt) and a temperature of about 30˚C. More specifically, the stringent conditions in microarray technique include the hybridization at 37˚C in 50% formamide, 1M NaCl and 1% SDS and washing at 60 to 65˚C in 0.1 x SSC. The stringent conditions in PCR technique include conditions of pH 7 to 9, 0.01 to 0.1 M Tris-HCl, 0.05 to 0.15 M potassium ion concentration (or other salt) and at least about 55˚C.

The sequence of the nucleic acid probe for detecting Th17 cells can be appropriately selected by a person skilled in the art based on the common technical knowledge in the art and the sequence of the polynucleotide marker so that it can specifically hybridize to the polynucleotide marker.

The nucleic acid probe for detecting Th17 cells can be designed by using, for example, a commonly available primer designing software (e.g. Primer3 (available from http://frodo.wi.mit.edu/cgi-bin/primer3/primer3.cgi) or DNASIS Pro (Hitachi Software Engineering Co., Ltd.)).

The nucleic acid probe for detecting Th17 cells can be prepared according to polynucleotide synthesis methods which are well-known in the art.

The nucleic acid probe for detecting Th 17 cells may be labeled with a labeling substance normally used in the art. The labeled nucleic acid probe for detecting Th 17 cells allows an easy detection of the polynucleotide marker for detecting Th17 cells, namely of Th17 cells.

The labeling substance may be a labeling substance generally used in the art including radioisotopes such as $^{32}P$, fluorescent substances such as fluorescein, enzymes such as alkaline phosphatase and horseradish peroxidase, and biotin.

Th17 cells can be specifically detected by using one or more nucleic acid probes for detecting Th17 cells. For example, a DNA chip or microarray for detecting the polynucleotide marker for detecting Th 17 cells can be obtained by fixing one or more probes on a substrate according to a method well-known in the art.

The nucleic acid probe for detecting Th 17 cells may include a set of two or more primers for amplifying the polynucleotide marker by PCR technique, for example.

A molecule that can specifically bind to the protein marker can be used for the detection of the marker, making it useful in the detection of Th17 cells. Such molecule may be a nucleic acid aptamer such as DNA or RNA or an antibody that can specifically bind to the protein marker, and preferably an antibody. When the marker specific for Th17 cells is an enzyme, it can be detected by applying a substrate for the enzyme to develop color or emit light or fluorescent.

The antibody for detecting Th17 cells can be prepared by the following well-known procedure, for example. A DNA molecule encoding a protein having an amino acid sequence of the protein marker is prepared based on the nucleic acid sequence of the polynucleotide marker or the amino acid sequence of the protein marker, and is introduced into an appropriate expression vector. The obtained expression vector is introduced into an appropriate host cell, and the obtained transformed cells are cultured to obtain a desired protein. The obtained protein is purified and used as an immunogen optionally with an adjuvant to immunize an appropriate mammal such as rat or mouse. Spleen cells of the immunized animals are screened for antibody producing cells that produce an antibody directed to the target immunogen. The selected antibody producing cells are fused with myeloma cells to obtain hybridomas. These hybridomas are screened for antibody producing hybridomas that produce an antibody having specific binding property to the protein encoded by the gene. The desired antibody can be obtained by culturing the obtained antibody producing hybridomas.

The nucleic acid aptamer that can be used for detecting Th 17 cells can be prepared by the following well-known procedure, for example. A nucleic acid library including random nucleic acid sequences is prepared according to the known technique, and an aptamer that specifically binds to the target protein (the protein marker) can be selected by the systematic evolution of ligands by exponential enrichment method (SELEX method) or the like.

The molecule which can specifically bind to the protein marker for detecting Th17 cells may be labeled with a labeling substance normally used in the art. The labeled antibody for detecting Th17 cells allows an easy detection of the protein marker for detecting Th17 cells, namely of Th17 cells.

The labeling substance may be a labeling substance generally used in the art including radioisotopes such as $^{32}P$, fluorescent substances such as fluorescein, enzymes such as alkaline phosphatase and horseradish peroxidase, and biotin.

The present invention also provides a method for detecting Th17 cells by detecting the presence of the polynucleotide or protein marker for detecting Th17 cells in a sample containing cells.

In the present method, the sample containing cells includes a biological sample obtained from mammals or a sample containing cultured cells. The biological sample includes blood, tissue, synovial fluid, cerebrospinal fluid, pleural fluid, ascitic fluid and the like.

An embodiment of the method for detecting the presence of the polynucleotide marker is described. Nucleic acid (DNA or RNA) is extracted from a sample containing cells by a well-known method in the art such as the one using a phenolic extraction and ethanol precipitation or a commercial DNA extraction kit.

Then, the presence of the polynucleotide marker in the obtained nucleic acid sample is detected, preferably using the nucleic acid probe for detecting Th17 cells.

The polynucleotide marker can be detected by well-known methods in the art including nucleic acid amplification methods such as PCR, RT-PCT, real-time PCR, loop-mediated isothermal amplification (LAMP), hybridization methods such as Southern hybridization, Northern hybridization, fluorescence in situ hybridization (FISH), DNA chip or microarray. Such methods are carried out under stringent conditions, and the hybridization of the nucleic acid probe for detecting Th17 cells is detected by detecting the labeling substance and the like to detect the presence of the polynucleotide marker.

An embodiment of the method for detecting the presence of the protein marker for detecting Th17 cells is described. When the target protein marker is an intracellular protein, it is extracted from cells by using well-known methods in the art. The extraction of the protein from cells can be accomplished by known methods such as disruption of the cells by ultrasonic, lysis of the cells with a cell lysis solution. The protein marker in the obtained protein sample can be detected by using the molecule which specifically binds to the protein marker. Specifically, the protein marker can be detected by well-known methods in the art such as enzyme linked immunosorbent assay (ELISA) or Western blotting. The molecule which specifically binds to the protein marker in the detection is preferably the above antibody for detecting Th17 cells.

When the target protein marker is a secretory protein, the protein marker secreted in the sample containing the cells can be detected by using the molecule which specifically binds to the protein marker. Alternatively, the cells (lymphocytes) are recovered from the sample and the obtained cells are stimulated with anti-CD3 antibodies, anti-CD28 antibodies, concanavalin A, phytohemagglutinin (PHA), phorbol myristate acetate (PMA), ionomycin or the like. Then, the secreted protein marker can be detected by using the molecule which specifically binds to the protein marker. Specifically, the protein marker can be detected by well-known methods in the art such as ELISA or Western blotting. The molecule which specifically binds to the protein marker in the detection is preferably the above antibody for detecting Th17 cells.

When the target protein marker is a protein located on the cell surface, the protein marker located on the cell surface in the sample containing the cells can be detected by using the molecule which specifically binds to the protein marker. Alternatively, a membrane fraction of the cells is obtained from the sample and the protein marker in the membrane fraction can be detected by using the molecule which specifically binds to the protein marker. Specifically, the protein marker can be detected by well-known methods in the art such as ELISA or Western blotting. When the target protein marker is a protein located on the cell surface, it can be detected by a method based on flow cytometry (FCM). The molecule which specifically binds to the protein marker in the detection is preferably the above antibody for detecting Th17 cells.

For example, the protein marker can be detected by FCM as follows.

First, the sample containing the cells is brought into contact with the antibody for detecting Th 17 cells labeled with an appropriate labeling substance. Th17 cells, when existing, bind the labeled antibody to their surfaces. Then, the sample containing the cells bound to the labeling substance can be applied to a flow cytometer to detect Th17 cells. Th17 cells that are bound to the labeling substance can optionally be classified and fractionated by using a cell sorter.

Such method of FCM is well-known to a person skilled in the art, who can appropriately select the reaction conditions.

## EXAMPLES

The present invention is now described in further detail. However, it is not intended that these Examples are to limit the scope of the present invention.

Example 1: Analysis of highly expressed genes in cultured Th17 cells derived from mice

1. Isolation of naïve T-cells from mouse spleen

The spleen was removed from BALB/c mice to obtain the sample containing spleen cells. Erythrocytes in the sample were lysed with ammonium chloride, and then cell fractions of CD8, B-cells, monocytes, macrophages, granulocytes and erythroblasts were removed from the sample by using magnetic beads (Polyscience) to partially purify $CD4^+$ T-cells. Sorting by a flow cytometer allowed the purification of naïve T-cell fraction ($CD4^+$/CD25neg/CD44low/CD62high) from $CD4^+$ T-cells. In a similar manner, naïve T-cells were purified from spleen cells of C57/BL6 mice.

2. Differentiation culture from naïve T-cells to Th1, Th2, Treg and Th17 cells

Naïve T-cells derived from BALB/c mice as obtained in the above 1. were inoculated in a 24-well plate coated with anti-CD3 antibody with the cell density of 0.5 to 2.0 x $10^6$ cells/2 ml/well. Cells were incubated in T-cell medium (PRMI1640, 10% fetal bovine serum (FBS), 10 mM HEPES, 1 mM sodium pyruvate, 2 mM L-glutamic acid, 50$\mu$M 2-mercaptoethanol, 100 U/ml penicillin, 100 mg/ml streptomycin) supplemented with cytokines and antibodies specified in Table 1 and anti-CD28 antibody in an incubator at 37°C, 5% $CO_2$. After 3 days from the initiation of the culture, cytokines and antibodies specified in Table 1 were added to the medium and the culture was continued for additional 2 to 11 days. Accordingly, the differentiation was induced from naïve T-cells derived from BALB/c mice to Th1, Th2, Treg and Th17 cells. In a similar manner, the differentiation was induced from naïve T-cells derived from C57/BL6 mice to Th1, Th2, Treg and Th17 cells.

[Table 1]

| | Cytokine | Manufacturer | Antibody | Clone | Manufacturer |
|---|---|---|---|---|---|
| Th1 cells | IL-2 | Becton Dickinson (Hereinafter, BD) | Anti-IL-4 antibody | 11B11 | eBioscience |
| | IL-12 | BD | | | |
| Th2 cells | IL-2 | BD | Anti-IFNγantibody | R4-6A2 | eBioscience |
| | IL-4 | R&D SYSTEM | | | |
| Treg cells | IL-2 | BD | Anti-IL-6 antibody | MP5-20F3 | BD |
| | TGFβ | R&D SYSTEM | Anti-IFNγantibody | R4-6A2 | eBioscience |
| | | | Anti-IL-4 antibody | 11B11 | eBioscience |

(continued)

| | Cytokine | Manufacturer | Antibody | Clone | Manufacturer |
|---|---|---|---|---|---|
| Th17 cells | IL-6 | BD | Anti-IL-2 antibody | S4B6 | BD |
| | TGFβ | R&D SYSTEM | Anti-IFNγ antibody | R4-6A2 | eBioscience |
| | IL-23 | R&D SYSTEM | Anti-IL-4 antibody | 11B11 | ebioscience |
| | IL-1β | eBioscience | | | |
| | TNFα | eBioscience | | | |

3. Confirmation of cell differentiation by flow cytometer

Phorbol myristate acetate (PMA, 50 ng/ml) and ionomycin (1μM) were added to the solution containing the cells (2.5 x $10^5$ cells) differentiated and cultivated as described in under point 2 above, to stimulate the cells. Brefeldin A (10μg/ml) was added after 4 hours and incubated for further 2 hours. After the incubation, cells were washed with phosphate buffered saline (PBS) and fixed with 4% paraformaldehyde. After the fixation, cells were treated with the saponin buffer (0.5% saponin, 0.5% BSA, 1 mM sodium azide, in PBS) to increase the permeability of the cell membrane. Then, cells were reacted with anti-IFN-γ, anti-IL-4 or anti-IL-17 antibodies. After the reaction, cells were washed with the saponin buffer and PBS containing 0.5% bovine serum albumin (BSA), and analyzed on FACS Canto II (BD Biosciences) to confirm the differentiation to Th1, Th2, Treg and Th17 cells, respectively.

4. Preparation of total RNA

Th1, Th2, Treg and Th17 cells derived from BALB/c mice cultured for 5 days as described under point 2 above, were respectively washed with PBS, centrifuged to pellets, and stored at -80°C. Total RNA was prepared from pellets by using RNeasy Plus Mini Kit (QIAGEN) and stored at -80°C until the analysis. In a similar manner, total RNA wase prepared respectively from Th1, Th2, Treg and Th17 cells derived from C57/BL6 mice cultured for 5 days as described in 2.

5. Expression analysis on microarray

Total RNA (1 to 5μg) prepared as described above under point 4, was reverse-transcribed to cDNA with One-Cycle Target Labeling and Control Reagents (Affymetrix) and further transcribed into biotinylated cRNA. Biotinylated cRNA (15μg) was added to GeneChip Mouse Genome 430 2.0 Array (Affymetrix), and hybridization was carried out in GeneChip Hybridization Oven 640 (Affymetrix) at the conditions of 45°C and 60 rpm for 16 hours. After the hybridization, the microarray (DNA chip) was washed and fluorescence labeled in GeneChip Fluidic Station 450 (Affymetrix) and scanned on GeneChip Scanner 3000 7G (Affymetrix) to obtain the fluorescent intensity data.

6. Selection of genes specifically expressed in mouse Th17 cells

Based on the fluorescent intensity data obtained as described under point 5 above, data were standardized with the expression analysis software Array Assist (MediBic). The fluorescent intensity of each gene was divided by the fluorescent intensity of the house keeping gene of glyceraldehyde-3-phosphate dehydrogenase (GAPDH) to calculate the relative fluorescent intensity of each gene. The relative fluorescent intensity of Th17 cells was compared with the relative fluorescent intensities of Th1, Th2 and Treg cells. The genes whose relative fluorescent intensities in Th17 cells were three times or more any of those of Th1, Th2 and Treg cells in at least one of BALB/c and C57/BL6 mice were identified as the genes which are specifically expressed in Th 17 cells (polynucleotide markers for detecting Th 17 cells). The identified genes are specified in Table 2 below.
Table 2 shows Probe Set IDs from Affimetrix, Unigene codes corresponding to Probe Set IDs, gene titles corresponding to Probe Set IDs of the respective genes and functions of the proteins encoded by the genes. Table 2 further shows the ratios of the relative fluorescent intensities in Th17 cells to those in Th1, Th2 and Treg cells, respectively, in BALB/c or C57/BL6 mice (Th17/Th1, Th17/Th2 and Th17/Treg, respectively).
Table 2 further shows NCBI codes representing the amino acid sequences of the proteins encoded by the genes.
[Table 2-1]

| Affimetrix Probe Set ID | Unigene code | NCBI code | Gene title | Function of encoded protein | Ratio of relative fluorescent intensity | | |
|---|---|---|---|---|---|---|---|
| | | | | | Th17/Th1 | Th17/Th2 | Th17/Treg |
| 1421672_at | Mm.5419 | NP_034682 | Interleukin 17A | Cytokine | 18763.8 | 104.1 | 119.5 |
| 1427624_s_at | Mm.103585 | NP_058667<br>NP_473420 | Interleukin 22, Interleukin tifb | Cytokine | 85.4 | 53.8 | 96.6 |
| 1422029_at | Mm.116739 | NP_058656 | Chemokine, CC motif, ligand 20 | Chemokine | 75.9 | 65.5 | 43.9 |
| 1426566_s_at | Mm.131781 | NP_001029201<br>NP_001029203<br>NP_665825 | Interleukin 17 receptor E | Membrane protein | 606.9 | 88.2 | 23.8 |
| 1448950_at | Mm.896 | NP_001116854<br>NP_032388 | Interleukin 1 receptor 1 | Membrane protein | 52.4 | 26.2 | 47.8 |
| 1449508_at | Mm.38386 | NP_057880 | Interleukin 27 receptor A | Membrane protein | 3.7 | 5.0 | 3.0 |
| 1431296_at | Mm.390873<br>Mm.426544 | XP_156321<br>XP_921879 | G protein-coupled receptor 15 | Membrane protein | 361.5 | 865.2 | 5.8 |
| 1450199_a_at | Mm.220821 | NP_619613 | Stabilin 1 | Membrane protein | 18.1 | 11.3 | 4.7 |
| 1419309_at | Mm.2976 | NP_034459 | Podoplanin | Membrane protein | 21.4 | 18.3 | 11.3 |
| 1419498_at | Mm.25138 | NP_079931 | Transmembrane and immunoglobulin domain containing 1 | Membrane protein | 36.4 | 21.2 | 9.9 |
| 1422926_at | Mm.426053 | NP_032586 | Melanocortin 2 receptor | Membrane protein | 67.5 | 5.0 | 77.0 |
| 1423909_at | Mm.27061 | NP_ 001091741<br>NP_079602 | 001091741 Transmembrane protein 176A | Membrane protein | 46.1 | 30.9 | 4.3 |
| 1428958_at | Mm.40780 | NP_083105 | Progestin and adipoQ receptor family member VIII | Membrane protein | 12.1 | 7.6 | 3.3 |
| 1437399_at | Mm.29482 | NP_741968 | Claudin domain containing 1 | Membrane protein | 39.9 | 15.6 | 3.0 |
| 1441891_x_at | Mm.286127 | NP_083277 | ELOVL family member 7, elongation of long chain fatty acids | Membrane protein | 5.7 | 319.1 | 7.2 |

(continued)

| Affimetrix Probe Set ID | Unigene code | NCBI code | Gene title | Function of encoded protein | Ratio of relative fluorescent intensity | | |
|---|---|---|---|---|---|---|---|
| | | | | | Th17/Th1 | Th17/Th2 | Th17/Treg |
| 1452855_at | Mm.273319 | NP_083903 | Lymphocyte antigen 6 complex, locus K | Membrane protein | 5.9 | 8.8 | 3.6 |
| 1457691_at | Mm.265618 | NP_898852 | G protein-coupled receptor 183 (Epstein-Barr virus induced gene 2) | Membrane protein | 4.1 | 35.6 | 8.7 |
| 1457722_at | Mm.259262 | NP_694734<br>NP_851409 | Killer cell lectin-like receptor subfamily B member 1F | Membrane protein | 10.1 | 38.0 | 6.4 |
| 1459994_x_at | Mm.21757 | NP_056614 | Transferrin receptor 2 | Membrane protein | 8.7 | 6.2 | 8.2 |
| 1416107_at | Mm.3304 | NP_032767 | Neuron specific gene family member 2 | Membrane protein | 7.5 | 6.6 | 9.1 |
| 1418004_a_at | Mm.28385 | NP_075543 | Transmembrane protein 176B | Membrane protein | 19.8 | 19.0 | 3.5 |
| 1421889_a_at | Mm.19133 | NP_001095925<br>NP_001095926<br>NP_033821 | Amyloid beta (A4) precursor-like protein 2 | Membrane protein | 4.3 | 7.9 | 3.1 |
| 1424305_at | Mm.1192 | NP_690052 | Immunoglobulin joining chain | Membrane protein | 10.5 | 298.6 | 7.4 |
| 1434601_at | Mm.24005 | NP_835215 | Adhesion molecule with Ig like domain 2 | Membrane protein | 5.8 | 17.8 | 4.4 |
| 1435477_s_at | Mm.425062 | NP_001070657<br>NP_034317 | Fc receptor, IgG, low affinity IIb | Membrane protein | 4.2 | 125.1 | 11.1 |
| 1450476_at | Mm.297251 | NP_034054 | Cannabinoid receptor 2 | Membrane protein | 9.3 | 4.8 | 7.4 |
| 1452425_at | Mm.215147 | NP_849262 | Tumor necrosis factor receptor superfamily, member 14 | Membrane protein | 4.0 | 6.5 | 4.0 |
| 1422007_at | Mm.34043 | NP_057898 | Aquaporin 3 | Membrane protein | 32.9 | 101.5 | 3.6 |
| 1422606_at | Mm.280158 | NP_112150 | C1q and tumor necrosis factor related protein 3 | Membrane protein | 78.8 | 26.7 | 42.3 |
| 1429314_at | Mm.379376 | NP_061274 | Synaptotagmin XI | Membrane protein | 3.5 | 18.9 | 6.3 |

(continued)

| Affimetrix Probe Set ID | Unigene code | NCBI code | Gene title | Function of encoded protein | Ratio of relative fluorescent intensity | | |
|---|---|---|---|---|---|---|---|
| | | | | | Th17/Th1 | Th17/Th2 | Th17/Treg |
| 1434881_s_at | Mm.246466 | NP_808383 | Potassium channel tetramerisation domain containing 12 | Membrane protein | 56.4 | 47.1 | 8.2 |
| 1436271 at | Mm.440965 | NP_001020019 | Apolipoprotein L 7b (expressed sequence BC085284), | Membrane protein | 271.8 | 21.8 | 3.3 |
| | Mm. 303207 | XP_997554 | Apolipoprotein L7e Similar to apolipoprotein L, 3) | | | | |

[Table 2-2]

| Affymetrix Probe Set ID | Unigene Code | NCBI Code | Gene title | Function of encoded protein | Ratio of relative fluorescent intensity | | |
|---|---|---|---|---|---|---|---|
| | | | | | Th17/Th1 | Th17/Th2 | Th17/Treg |
| 1439519_at | Mm.346652 | NP_543130 | Solute carrier family 34 (sodium phosphate), member 3 | Membrane protein | 13.0 | 15.4 | 4.2 |
| 1448754_at | Mm.279741 | NP_035384 | Retinol binding protein 1, cellular | Membrane protein | 24.8 | 28.1 | 11.8 |
| | | XP_001473672 | similar to cellular retinol binding protein I | | | | |
| 1449471_at | Mm.440652 | NP_067427.1 | Potassium large conductance calcium-activated channel, subfamily M, beta member 4, similar to calcium activated potassium channel beta 4 subunit | Membrane protein | 3.4 | 6.0 | 3.1 |
| 1450057_at | Mm.44218 | NP_079851 | SYS1 Golgi-localized integral membrane protein homolog (RIKEN cONA 2610042014 gene) (RIKEN cDNA 2610042014 gene) | Membrane protein | 3.4 | 5.1 | 4.2 |
| 1456464_x_at | Mm.379376 | NP_061274 | Synaptotagmin XI | Membrane protein | 3.9 | 13.8 | 5.0 |
| 1457266_at | Mum.290605 Mm. 290605 | XP_921985 | Solute carrier family 38, member 6 XP_988301 (expressed sequence AW322671) ( expressed sequence AW322671) | Membrane protein | 4.0 | 5.3 | 3.3 |
| | | XP_988301 | | | | | |
| 1416957_at | Mm.897 | NP_035266 | POU domain, class 2, associating factor 1 | Transcription/translation factor | 8.9 | 26.2 | 8.5 |

(continued)

| Affymetrix Probe Set ID | Unigene Code | NCBI Code | Gene title | Function of encoded protein | Ratio of relative fluorescent intensity | | |
|---|---|---|---|---|---|---|---|
| | | | | | Th17/Th1 | Th17/Th2 | Th17/Treg |
| 1433471_at | Mm.31630 | NP_033357 | Transcription factor 7 (T-cell specific) | Transcription/translation factor | 18.1 | 31.4 | 5.8 |
| 1437155_a_at | Mm.405029 | NP_598545 | WW domain containing transcription regulator 1 | Transcription/translation factor | 3.4 | 73.4 | 18.4 |
| 1443161_at | Mm.30466 | NP_114389 | Trichorhinophalangeal syndrome I | Transcription/translation factor | 18.6 | 7.8 | 3.5 |
| 1425642_at | Mm.229114 | NP_666121 | Centrosomal protein 290 | Transcription/translation factor | 83.3 | 527.5 | 3.5 |
| 1418314_a_at | Mm.370334 | NP_067452<br>NP_899011 | Ataxin 2 binding protein 1 | Transcription/translation factor | 228.6 | 43.1 | 4.7 |
| 1422562_at | Mm.29467 | NP_062636 | Ras-related associated with diabetes | Signaling molecule | 3.4 | 6.5 | 4.7 |
| 1415936_at | Mm.45815 | NP_038895 | Breast cancer anti-estrogen resistance 3 | Signaling molecule | 288.7 | 17.7 | 3.7 |
| 1417700_at | Mm.276669 | NP_082514 | Rab38, member of RAS oncogene family | Signaling molecule | 7.6 | 18.2 | 5.6 |
| 1435432_at | Mm.291135 | NP_001032213<br>NP_835220 | Centaurin, gamma 2 | Signaling molecule | 97.6 | 16.7 | 3.1 |
| 1435644_at | Mm.227616 | NP_796338 | SH3 and PX domains 2B | Signaling molecule | 27.5 | 19.2 | 10.0 |
| 1440799_s_at | Mm.243091 | NP_663494 | FERM, RhoGEF and pleckstrin domain protein 2 | Signaling molecule | 8.4 | 29.2 | 5.8 |
| 1420498_a_at | Mm.240830 | NP_001008702<br>NP_001032994<br>NP_001095870<br>NP_075607 | Disabled homolog 2 | Signaling molecule | 74.0 | 195.0 | 52.3 |

(continued)

| Affymetrix Probe Set ID | Unigene Code | NCBI Code | Gene title | Function of encoded protein | Ratio of relative fluorescent intensity | | |
|---|---|---|---|---|---|---|---|
| | | | | | Th17/Th1 | Th17/Th2 | Th17/Treg |
| 1419004_s_at | Mm.378888 | NP_031560 | B-cell leukemia/ lymphoma 2 related protein A1b, | Signaling molecule | 3.6 | 17.7 | 8.7 |
| | | NP_031562 | B-cell leukemia/ lymphoma 2 related protein A1d, | | | | |
| | | NP_033872 | B-cell leukemia/ lymphoma 2 related protein A1a | | | | |
| 1415871_at | Mm.14455 | NP_033395 | Transforming growth factor, beta induced | Adhesion molecule | 91.1 | 55.2 | 84.0 |
| 1416612_at | Mm.214016 | NP_034124 | Cytochrome P450, family 1, subfamily b, polypeptide 1 | Enzyme | 12.9 | 4.0 | 23.5 |
| 1417235_at | Mm.18526 | NP_065603 | EH-domain containing 3 | Enzyme | 4.0 | 20.8 | 3.4 |
| 1417256_at | Mm.5022 | NP_032633 | Matrix metallopeptidase 13 | Enzyme | 13.9 | 12.6 | 5.3 |
| 1418018_at | Mm.276736 | NP_031780 | Carboxypeptidase D | Enzyme | 87.1 1 | 27.3 | 6.1 |
| 1421307_at | Mm.158776 | NP_078771 | Carbonic anhydrase 13 | Enzyme | 9.2 | 7.4 | 9.5 |
| 1421415_s_at | Mm.314757 | NP_032131 | Glucosaminyl (N-acetyl) transferase 2, I-branching enzyme | Enzyme | 15.4 | 5.2 | 13.2 |
| | | NP_076376 | | | | | |
| | | NP_573482 | | | | | |

[Table 2-3]

| Affymetrix Probe Set ID | Unigene Code | NCBI Code | Gene title | Function of encoded protein | Ratio of relative fluorescence intensity | | |
|---|---|---|---|---|---|---|---|
| | | | | | Th17/Th1 | Th17/Th2 | Th17/Treg |
| 1424783_a_at | Mm.300095 | NP_038729 | UDP glucuronosyltransferase 1 family, polypeptide A2, | Enzyme | 12.7 | 183.1 | 4.2 |
| | | NP_659545 | UDP glucuronosyltransferase 1 family, polypeptide A6A, | | | | |
| | | NP_958812 | UDP glucuronosyltransferase 1 family, polypeptide A6B, | | | | |
| | | NP_964003 | UDP glucuronosyltransferase 1 family, polypeptide A10, | | | | |
| | | NP_964004 | UDP glucuronosyltransferase 1 family, polypeptide A7C, | | | | |
| | | NP_964005 | UDP glucuronosyltransferase 1 family, polypeptide A5, | | | | |
| | | NP_964006 | UDP glucuronosyltransferase 1 family, polypeptide A9, | | | | |
| | | NP_964007 | UDP glucuronosyltransferase 1 family, polypeptide A1, | | | | |
| | | XP_911442 | similar to UDP glycosyltransferase 1 family, polypeptide A8 | | | | |
| 1425128_at | Mm.192369 | NP_001031817 | UDP-GlcNAc:betaGal beta-1,3-N-acetylglucosaminyltransferase 8 | Enzyme | 7.4 | 10.0 | 7.8 |
| | | NP_666296 | | | | | |
| 1426238_at | Mm.27757 | NP_033885 | Bone morphogenetic protein 1 | Enzyme | 3.5 | 12.5 | 7.9 |
| 1448562_at | Mm.4610 | NP_033503 | Uridine phosphorylase 1 | Enzyme | 38.8 | 9.2 | 8.1 |
| 1459299_at | Mm.99648 | NP_796350 | Myosin IIIB | Enzyme | 8.7 | 11.7 | 4.4 |
| 1416673_at | Mm.97885 | NP_062390 | Beta-site APP-cleaving enzyme 2 | Enzyme | 6.0 | 4.1 | 15.2 |
| 1422352_at | Mm.201549 | NP_032596 | Mast cell protease 1 | Enzyme | 39.8 | 30.3 | 15.8 |

(continued)

| Affymetrix Probe Set ID | Unigene Code | NCBI Code | Gene title | Function of encoded protein | Ratio of relative fluorescence intensity | | |
|---|---|---|---|---|---|---|---|
| | | | | | Th17/Th1 | Th17/Th2 | Th17/Treg |
| 1429329_at | Mm.340211 | NP_848466 | COX10 homolog, cytochrome oxidase assembly protein, heme A: farnesyltransferase | Enzyme | 3.2 | 3.1 | 3.2 |
| 1438801_at | Mm.441620 | NP_001033708<br>NP_766234 | Dynamin 3 | Enzyme | 17.3 | 9.3 | 3.1 |
| 1441975_at | Mm.19941 | NP_062781<br>NP_997551 | Acid phosphatase, prostate | Enzyme | 10.6 | 6.9 | 14.6 |
| 1445963_at | Mm.134911 | NP_700471 | Phosphodiesterase 5A, cGMP-specific | Enzyme | 74.8 | 65.7 | 141.5 |
| 1451361_a_at | Mm.389243 | NP_666363 | Patatin-like phospholipase domain containing 7 | Enzyme | 3.7 | 4.9 | 4.5 |
| 1453474_at | Mm.432526 | NP_080461 | RIKEN cDNA 1300007F04 gene | Enzyme | 17.5 | 16.2 | 4.3 |
| 1454013_at | Mm.437061 | NP_084456 | RIKEN cDNA 1810062018 gene | Enzyme | 12.4 | 121.6 | 3.9 |
| 1457063_at | Mm.133075 | NP_694744 | Phosphatase, orphan 1 (expressed sequence A1447357, ABI gene family, member 3) | Enzyme | 5.8 | 10.3 | 4.3 |
| 1458296_at | Mm.309395 | NP_034292 | Exostoses (multiple) 1 | Enzyme | 3.6 | 13.1 1 | 3.9 |
| 1416318_at | Mm.20144 | NP_079705 | Serine (or cysteine) peptidase inhibitor, clade B, member 1a | Enzyme inhibitor | 9.4 | 284.5 | 40.9 |
| 1417701_at | Mm.308126 | NP_597844 | Protein phosphatase 1, regulatory (inhibitor) subunit 14c | Enzyme inhibitor | 6.6 | 3.9 | 6.0 |
| 1421137_a_at | Mm.262135 | NP_001034139<br>NP_001034140<br>NP_001034141<br>NP_001034142<br>NP_032889 | Protein kinase inhibitor beta, cAMP dependent, testis Protein kinase inhibitor beta, cAMP dependent, testis specific | Enzyme inhibitor | 5.9 | 17.9 | 5.8 |

(continued)

| Affymetrix Probe Set ID | Unigene Code | NCBI Code | Gene title | Function of encoded protein | Ratio of relative fluorescence intensity | | |
|---|---|---|---|---|---|---|---|
| | | | | | Th17/Th1 | Th17/Th2 | Th17/Treg |
| 1460227_at | Mm.8245 | NP_001037849<br>NP_035723 | Tissue inhibitor of metalloproteinase 1 | Enzyme inhibitor | 17.1 | 39.4 | 182.4 |
| 1416702_at | Mm.41560 | NP_033276 | Serine (or cysteine) peptidase inhibitor, clade I, member 1 | Enzyme inhibitor | 5.8 | 20.5 | 3.4 |
| 1420621_a_at | Mm.277585 | NP_031497 | Amyloid beta (A4) precursor protein | Enzyme inhibitor | 3.4 | 4.0 | 7.0 |
| 1424351_at | Mm.27289 | NP_080599 | WAP four-disulfide core domain 2 | Enzyme inhibitor | 30.7 | 35.5 | 130.2 |
| 1434758_at | Mm.264680 | NP_084485 | Cysteine-rich secretory protein LCCL domain containing 2 | Secretory protein | 294.1 | 432.9 | 42.7 |

[Table 2-4]

| Affymetrix Probe Set ID | Unigene code | NCBI code | Gene title | Function of encoded protein | Ratio of relative fluorescence intensity | | |
|---|---|---|---|---|---|---|---|
| | | | | | Th17/Th1 | Th17/Th2 | Th17/Treg |
| 1460406_at | Mm. 11869 | NP_ 001028382 | Plastin 1 ( expressed sequence AI427122) | Structural protein | 8.2 | 8.9 | 7.1 |
| 1421653_a_ at | Mm. 246497 | XP_ 990954 | Immunoglobulin heavy chain complex, Immunoglobulin heavy chain 1a (serum IgG2a), Immunoglobulin heavy chain 2 (serum IgA), Immunoglobulin heavy chain Ia, Immunoglobulin heavy chain (J558 family), Immunoglobulin heavy chain (gamma polypeptide), similar to immunoglobulin mu-chain, similar to immunoglobulin heavy chain V region3 precursor, Immunoglobulin heavy chain variable region, similar to immunoglobulin heavy chain V region 102 precursor | Structural protein | 496.7 | 96.2 | 4.1 |
| 1424631_a_ at | Mm. 436014 | XP_ 001472591 | Immunoglobulin heavy chain (gamma polypeptide) | Structural protein | 32.0 | 6.0 | 20.8 |

(continued)

| Affymetrix Probe Set ID | Unigene code | NCBI code | Gene title | Function of encoded protein | Ratio of relative fluorescence intensity | | |
|---|---|---|---|---|---|---|---|
| | | | | | Th17/Th1 | Th17/Th2 | Th17/Treg |
| 1426174_s_at | Mm. 342177<br>Mem. 436014 | XP_001472591 | Immunoglobulin heavy chain 3,<br>Immunoglobulin heavy chain (gamma polypeptide) | Structural protein | 17.2 | 18.9 | 3.7 |
| 1438452_at | Mm. 256298 | NP_083033 | Nebulette | Structural protein | 36.9 | 14.8 | 6.9 |
| 1423607_at | Mm. 18888 | NP_032550 | Lumican | Structural protein | 5.0 | 70.2 | 39.9 |
| 1437232_at | Mm. 107214 | NP_808440 | Bactericidal/ permeability-increasing protein-like 2 | Structural protein | 4.5 | 9.5 | 4.6 |
| 1433526_at | Mm. 179871 | NP_848856 | Kelch-like 8 | Structural protein | 5.3 | 43.2 | 3.7 |
| 1459860_x_at | Mm. 44876 | NP_109631 | Tripartite motif-containing 2 | Structural protein | 12.4 | 21.0 | 7.8 |
| 1422862_at | Mm. 117709 | NP_062782<br>NP_062783<br>NP_072048 | PDZ and LIM domain 5 | Structural protein | 10.0 | 66.8 | 3.8 |
| 1427118_at | Mm. 347934 | NP_058575 | RIKEN cDNA 5430421 N21 gene | Structural protein | 48.8 | 7.1 | 3.5 |
| 1434947_at | Mm.7688 | NP_032471 | Kinesin family member 3C | Structural protein | 3.6 | 5.1 | 3.4 |
| 1423994_at | Mm. 402393 | NP_032467<br>NP_997565 | Kinesin family member 1B | Structural protein | 4.0 | 6.8 | 3.4 |
| 1455266_at | Mm. 256342 | NP_032475 | Kinesin family member 5C | Structural protein | 4.1 | 7.2 | 3.6 |

[Table 2-5]

| Affymetrix Probe Set ID | Unigene code | NCBI code | Gene title | Function of encoded protein | Ratio of relative fluorescent intensity | | |
|---|---|---|---|---|---|---|---|
| | | | | | Th17/Th1 | Th17/Th2 | Th17/Treg |
| 1427417_at | Mm. 98731 | NP_766526 | Sex comb on midleg-like 4 midleg-like | sex comb on 4 | 5.2 | 19.0 | 3.2 |
| 1440559_at | Mm. 441435 | NP_835158 | High mobility group AT-hook 2, pseudogene 1 | high mobility group AT-hook 2 | 20.2 | 4.9 | 7.4 |
| 1432280_at | Mm. 159539 | XP_126508<br>XP_918060 | RIKEN cDNA 2310007L24 gene | hypothetical protein LOC75573 isoform 1 | 180.6 | 54.1 | 10.3 |
| 1437145_s_at | Mm. 46431 | NP_080691 | RIKEN cDNA 2310002J15 gene | hypothetical protein LOC67859 | 74.5 | 34.4 | 11.1 |
| 1429764_at | mum. 34131 | XP_898485<br>XP_988635 | Family with sequence similarity 101, member B ( RIKEN cDNA 1500005K14 gene ) | hypothetical protein LOC76566 | 6.5 | 6.4 | 3.6 |
| 1456603_at | Mm. 34131 | XP_898485<br>XP_988635 | Family with sequence similarity 101, member B ( RIKEN cDNA 1500005K14 gene ) | hypothetical protein LOC76566 | 9.6 | 16.6 | 5.2 |
| 1456878_at | Mm. 259320 | NP_942560 | expressed sequence AI646023 LOC192734 | hypothetical protein | 5.2 | 20.2 | 5.3 |
| 1428736_at | Mm. 24356 | NP_08051 | GRAM domain containing 3 LOC107022 | hypothetical protein | 3.9 | 89 | 3.4 |
| 1440156_s_at | Mm. 207709 | NP_001092269 | TOX high mobility group box family member 2 ( expressed sequence AI851523 ) | EST | 76.8 | 5.9 | 3.3 |
| 1443078_at | Mm. 399703 | - | RIKEN cDNA 6030439D06 gene | EST | 8.3 | 42.7 | 13.0 |
| 1452952_at | Mm. 220761 | NP_001074758 | RIKEN cDNA 9030418K01 gene | EST | 25.3 | 8.8 | 9.3 |

(continued)

| Affymetrix Probe Set ID | Unigene code | NCBI code | Gene title | Function of encoded protein | Ratio of relative fluorescent intensity | | |
|---|---|---|---|---|---|---|---|
| | | | | | Th17/Th1 | Th17/Th2 | Th17/Treg |
| 1444674_at | Mm. 227443 | - | expressed sequence AU015680 | EST | 42.4 | 3.4 | 11.8 |
| 1457174_at | Mm. 227443 | - | expressed sequence AU015680 | EST | 8.2 | 4.3 | 3.9 |
| 1458341_x_ at | - | - | Polynucleotide SEQ ID NO:1 | EST | 13.9 | 29.7 | 15.4 |
| 1460118_at | Mm. 401203 | - | Polynucleotide SEQ ID NO:2 | EST | 11.7 | 14.3 | 8.6 |

The ratio of the relative fluorescent intensity for the gene RAR-related orphan receptor gamma is shown in Table 3, which is known to be specifically expressed in Th17 cells.

[Table 3]

| Affymetrix Probe Set ID | Unigene code | NCBI code | Gene title | Function of encoded protein | Ratio of relative fluorescent intensity | | |
|---|---|---|---|---|---|---|---|
| | | | | | Th17/ Th1 | Th17/ Th2 | Th17/ Treg |
| 1425792_a_ at | Mm.4372 | NP_ 035411 | RAR-related orphan receptor gamma | Transcription factor | 428.6 | 405.0 | 8.2 |

These results show that the genes specified in Table 2 are specifically expressed in Th17 cells as the gene specified in Table 3 which has been known to be specifically expressed in Th17 cells. The procedures described under the above points 1 to 5 were repeated four times, and it was confirmed that the relative fluorescent intensities of the above genes in Th17 cells were three times or more than any of those of Th1, Th2 and Treg cells.

Example 2: Analysis of highly expressed genes in disease mouse models

1. Generation of disease mouse models

1) Generation of SKG arthritis mouse model (hereinafter referred to as "arthritis model mice")

Arthritis model mice were generated according to the following procedures.

a) Preparation of bacterial cell components and administration to mice

Curdlan from *Alcaligenes faecalis* (SIGMA) was suspended in PBS to prepare a curdlan preparation (50 mg/ml) (hereinafter referred to as "bacterial cell components"). The bacterial cell components were intraperitoneally administered to 7 to 8 week-old female SKG spontaneously arthritis mice (Nature, vol 426, pp.454-460 (2003), purchased from CLEA Japan, Inc.) at 200μl/mouse. After four weeks, the bacterial cell components were further intraperitoneally administered at 200μl/mouse.

b) Evaluation of severity of arthritis

Severity was evaluated according to the following scores.

Score 0: Normal
Score 1: Mild joint inflammation
Score 2: Mild joint inflammation and swelling
Score 3: Moderate joint inflammation and swelling
Score 4: Severe joint inflammation and swelling
Score 5: Severe joint inflammation, swelling and joint deformity
Score 6: Severe joint inflammation, swelling, joint deformity, walking difficulty and debilitation

According to the above criterion, mice used for analysis were selected. Symptoms of arthritis appear at 30 days or more after the administration of the bacterial cell components. For the analysis, two mice evaluated as Score 0 at three weeks after the administration, two mice evaluated as Score 3 at eight weeks after the administration, two mice evaluated as Score 5 at twelve weeks after the administration (hereinafter referred to as "fastigium arthritis model mice"), two mice evaluated as Score 6 at twenty weeks after the administration and two mice evaluated as Score 0 at twenty weeks to which no bacterial cell component was administered (10 mice, in total). Control mice were two BALB/c mice (Oriental Yeast Co., Ltd.).

2) Generation of experimental allergic encephalomyelitis (EAE) (acute) mouse model (hereinafter referred to as "encephalitis model mice")

Encephalitis model mice were generated according to the following procedures.

a) Preparation of antigen emulsion and administration to mice

Incomplete Freund's adjuvant (Difco Laboratories) and the cell components of *Mycobacterium tuberculosis* H37Ra (Difco Laboratories) were mixed to obtain 40 mg/ml complete Freund's adjuvant (CFA). PLP (myelin proteolipid protein) peptide (positions 139 to 151, amino acid sequence: HSLGKWLGHPDKF, prepared by Hokkaido System Science Co., Ltd.) dissolved in PBS at 2 mg/ml was mixed with CFA in equal quantities in a syringe equipped with a double hub needle (Techno Chemical Corporation) to prepare an antigen emulsion. Female SJL mice (8 to 10-week old) (Charles River Laboratories Japan Inc.) were shaved at their back with hair clippers and subcutaneously administered with 50μl of the antigen emulsion using a 1-ml syringe at two positions, i.e. left and right sides of the midline of the waist of mice. On the next day of the injection, mice were administered with 200μl of Pertussis Toxin (List Biological Laboratories) dissolved in PBS (2μg/ml) by intravenous injection at the tail.

b) Evaluation of severity of encephalomyelitis

Severity was evaluated according to the following scores.

Score 0: Normal
Score 1: Tail paralysis
Score 2: Hind limb paresis
Score 3: Hind limb paralysis
Score 4: Forelimb paralysis
Score 5: Moribundity or death due to general paralysis

According to the above criterion, mice used for analysis were selected. Symptoms of encephalomyelitis appear at 10 to 14 days after the administration of the antigen emulsion. The symptoms are remitted and disappear at 15 to 20 days after the administration. For the analysis at the fastigium of the symptoms, five mice evaluated as Score 2 or more at 14 days after the administration (hereinafter referred to as "fastigium encephalitis model mice") were used. For the analysis at the remission of the symptoms, five mice evaluated as Score 0 at 18 days after the administration (hereinafter referred to as "remitted encephalitis model mice") were used (10 mice, in total). Control for encephalitis model mice were five SJL mice intraperitoneally administered with Pertussis Toxin only.

2. Preparation of Total RNA

1) Preparation of total RNA from tissues of arthritis model mice

Skin at the joint portions of arthritis model mice was removed with scissors, toes were separated and foot joint tissues were removed. The obtained foot joint tissues were frozen and stored in liquid nitrogen. Total RNA was prepared from the frozen foot joint tissues by using RNeasy Plus Mini kit (QIAGEN) and QIAshredder (QIAGEN). Total RNA from control mice was prepared in a similar manner.

2) Preparation of total RNA from tissues of encephalitis model mice

Encephalitis model mice were dissected to remove head and tail and spinal column was removed. PBS was injected from vertebral foramen of vertebrae coccygea of the spinal column and spinal cord was removed by injection pressure. The obtained spinal cord was frozen in liquid nitrogen. The frozen spinal cord tissue was homogenized with a homogenizer (AS ONE Corporation), and total RNA was prepared by using RNeasy Plus Mini kit (QIAGEN) and QIAshredder (QIAGEN). Total RNA from control mice was prepared in a similar manner.

3. Gene expression analysis in respective disease model mice on microarray

By using microarray, expression analysis of 115 genes was carried out in disease model mice which were selected as candidate markers for detecting Th17 cells. Total RNAs prepared from arthritis model mice, encephalitis model mice and control for each model mice were used in the analyses.
By using One-Cycle Target Labeling and Control Reagents (Affymetrix) or Two-Cycle Target Labeling and Control Reagents (Affymetrix), total RNAs (1 to 5μg for the One-cycle Reagents and 10 to 100μg for the Two-Cycle Reagents) were reverse-transcribed into cDNA, and then transcribed into biotinylated cRNA. Biotinylated cRNA (15μg) was placed in GeneChip Mouse Genome 430 2.0 Array (Affymetrix) and hybridization was carried out in GeneChip Hybridization Oven 640 (Affymetrix) at the conditions of 45°C and 60 rpm for 16 hours. After the hybridization, the microarray washed and fluorescent labeled in GeneChip Fluidic Station 450 (Affymetrix) was scanned in GeneChip Scanner 3000 7G (Affymetrix) to obtain the fluorescent intensity data.
The data were standardized with an expression analysis software Gene Spring GX (Agilent). Fluorescent intensity of each gene was divided by that of GAPDH to calculate the relative fluorescent intensity. The average values of the relative fluorescent intensities of disease model mice and control mice were calculated based on the number of mice used in the analyses. The average values correspond to the expression level of respective genes of the disease model mice and control mice in this Example.
In order to calculate the ratio of the expression of the genes of the disease model mice to the control mice, the expression levels of the disease model mice were divided by those of the corresponding control mice. The obtained values correspond to the ratio of the expression of the genes in arthritis and encephalitis model mice. For example, when the ratio of the expression of a gene is 2, it means that the expression level of the gene is two times higher in the disease model mice than in the control mice.

4. Identification of highly expressed gene in disease model mice 1) Identification according to ratio of expression of genes

The present inventors focused on the gene expression at the fastigium of the symptoms in the disease model mice. Namely, genes whose expression is increased at the fastigium were extracted based on the condition for genes highly expressed in the disease model mice (hereinafter referred to as "Condition 1"), being that the ratio of the expression of the genes at the fastigium to the normal state (in control mice) is 2 or more.
Among the genes which have been confirmed to be highly expressed in the cultured Th17 cells, the highly expressed genes in the fastigium arthritis model mice were identified according to the Condition 1. The results are shown in Table 4.
The highly expressed genes in the fastigium encephalitis model mice were also identified in a similar manner according to the Condition 1. The results are shown in Table 5.

2) Identification according to the correlation between expression levels of genes and that of IL-17A gene

The present inventors also focused on the kinetics of the expression level of IL-17A gene in disease model mice. Namely, genes whose expression level changed depending on the expression level of IL-17A were identified under the condition for genes correlating to the IL-17A gene expression (hereinafter referred to as "Condition 2"), being that the "Pearson product-moment correlation coefficient" is 0.6 or more between the expression level of the genes and that of IL-17A in disease model mice. In the art, the coefficient being 0.6 or more is believed to be statistically significant.
In the present Example, Pearson product-moment correlation coefficient was calculated as follows.
In a single disease model, we let the expression level of the gene for which the correlation is to be calculated and that of IL-17A gene be x and y, respectively. The i values of mice in the disease models were determined as follows.
In the arthritis model:

- i = 1 for control mice;
- i = 2 for the mice without the bacterial cell components administration; and
- i = 3 for the mice at three weeks after the administration of the bacterial cell components.

In the encephalitis model:

- i = 1 for control mice;
- i = 2 for the mice at 9 days after the administration of the antigen emulsion;
- i = 3 for the mice at 14 days after the administration of the antigen emulsion;
- i = 4 for the mice at 18 days after the administration of the antigen emulsion; and
- i = 5 for the mice at 24 days after the administration of the antigen emulsion.

The above defined values and an equation (x, y) = [(xi, yi)] (i = 1, 2, ... n) were used to obtain a data series consisting of two pairs of numeral values. In the arthritis model, n is 3 and in the encephalitis model, n is 5.
The following equation was used for the calculation of Pearson product-moment correlation coefficient. In the equation, x and y with overbar are the average values of x = {xi} and y = {$y_i$}, respectively.
[Formula 1]

$$\frac{\sum_{i=1}^{n}(x_i-\overline{x})(y_i-\overline{y})}{\sqrt{\sum_{i=1}^{n}(x_i-\overline{x})^2}\sqrt{\sum_{i=1}^{n}(y_i-\overline{y})^2}}$$

a) Arthritis model mice

The expression level of IL-17A gene in the arthritis model mice was increased at three weeks after the administration of the bacterial cell components at which period of time mice are evaluated as presymptomatic of Score O. Accordingly, Pearson product-moment correlation coefficients between the expression levels of IL-17A gene and the genes identified as described under point 4.1) above were calculated in the control mice, the mice without bacterial cell components administration and the arthritis model mice at three weeks after the administration of the bacterial cell components.
Based on the calculated coefficients and the Condition 2, the genes whose expressions correlate with that of IL-17A gene were identified in arthritis model mice. The results are shown in Table 4 with asterisks.
[Table 4-1]

| | Gene title | Affymetrix Probe Set ID | Expression level (vs. GAPDH) | | | | Ratio of expression (vs. control) | Correlation coefficient (vs. Il17a) |
|---|---|---|---|---|---|---|---|---|
| | | | Control mice | Arthritis model mice | | | Fastigium arthritis model mice (12w) | Balbc, 20w w/o bacteria admin., 3w after bacteria admin. |
| | | | | w/o bacteria admin. 20w | 3w | 12w (fastigium) | | |
| * | Interleukin 17A | 1421672_at | 207.8 | 226.3 | 1070.3 | 3819.4 | 18.4 | 1.0000 |
| * | Cysteine-rich secretory protein LCCL domain containing 2 | 1437056_x_at | 9461.9 | 11692.6 | 68955.5 | 42249.5 | 4.5 | 0.9999 |
| | | 1434758_at | 6190.9 | 4396.2 | 24640.2 | 17122.4 | 2.8 | 0.9951 |
| | | 1460458_at | 2411.9 | 1507.1 | 11033.9 | 9248.4 | 3.8 | 0.9945 |
| * | Tissue inhibitor of metalloproteinase 1 | 1460227_at | 5859.1 | 6598.9 | 19489.8 | 122655.6 | 20.9 | 0.9996 |
| | * Serine (or cysteine) peptidase inhibitor, clade B, member 1a | 1416318_at | 19872.6 | 20917.2 | 31810.5 | 59731.1 | 3.0 | 0.9982 |
| | | 1448301_s_at | 2171.7 | 3751.0 | 3690.4 | 11305.4 | 5.2 | 0.4868 |
| * | Matrix metallopeptidase 13 | 1417256_at | 6699.9 | 6086.5 | 17800.6 | 151299.2 | 22.6 | 0.9979 |
| * | Phosphodiesterase 5A (cGMP-specific) | 1445963_at | 72.2 | 60.3 | 188.1 | 468.3 | 6.5 | 0.9947 |
| * | C1q and tumor necrosis factor related protein 3 | 1422606_at | 631.3 | 341.8 | 1957.3 | 34652.4 | 54.9 | 0.9825 |
| * | Solute carrier family 38, member 6 ( expressed sequence AW322671 ) | 1457266_at | 5699.1 | 5825.5 | 6188.3 | 19804.9 | 3.5 | 0.9730 |
| * | RIKEN cDNA 9030418K01 gene | 1452952_at | 4179.0 | 4858.6 | 6652.8 | 13171.1 | 3.2 | 0.9688 |

(continued)

| | Gene title | Affymetrix Probe Set ID | Expression level (vs. GAPDH) | | | | Ratio of expression (vs. control) | Correlation coefficient (vs. Il17a) |
|---|---|---|---|---|---|---|---|---|
| | | | Control mice | Arthritis model mice | | | Fastigium arthritis model mice (12w) | Balbc, 20w w/o bacteria admin., 3w after bacteria admin. |
| | | | | w/o bacteria admin. 20w | 3w | 12w (fastigium) | | |
| * | Transmembrane protein 176A | 1441811_x_at | 5738.6 | 6814.2 | 9340.0 | 17201.0 | 3.0 | 0.9620 |
| | | 1423909_at | 5048.1 | 7214.1 | 8456.5 | 22824.8 | 4.5 | 0.7900 |
| | | 1425603_at | 4520.0 | 8194.4 | 9117.6 | 15556.4 | 3.4 | 0.6693 |
| * | Apolipoprotein L 7b ( expressed sequence BC085284 ), Apolipoprotein L7e ( similar to apolipoprotein L, 3 ) | 1436271_at | 67.0 | 97.9 | 163.0 | 355.0 | 5.3 | 0.9548 |
| * | Synaptotagmin XI | 1455176_a_at | 1336.4 | 1704.4 | 2095.5 | 4220.5 | 3.2 | 0.8836 |
| | | 1449264_at | 219.7 | 65.6 | 369.9 | 478.9 | 2.2 | 0.8526 |
| | | 1429314_at | 371.6 | 750.7 | 1035.0 | 1492.9 | 4.0 | 0.8325 |
| * | Retinol binding protein 1, cellular similar to cellular retinol binding protein | 1448754_at | 2393.6 | 1377.7 | 3518.6 | 9143.3 | 3.8 | 0.8713 |
| * | Lumican | 1423607_at | 45156.4 | 25541.4 | 61678.3 | 172983.7 | 3.8 | 0.8300 |
| * | Interleukin 1 receptor 1 | 1448950_at | 5275.9 | 3516.4 | 6544.6 | 33022.5 | 6.3 | 0.8047 |
| * | Kinesin family member 5C | 1455266_at | 1471.4 | 1839.0 | 2064.2 | 4452.2 | 3.0 | 0.8005 |
| * | Cannabinoid receptor 2 | 1450476_at | 596.9 | 1315.6 | 1580.7 | 3200.9 | 5.4 | 0.7214 |

(continued)

| | Gene title | Affymetrix Probe Set ID | Expression level (vs. GAPDH) | | | | Ratio of expression (vs. control) | Correlation coefficient (vs. Il17a) |
|---|---|---|---|---|---|---|---|---|
| | | | Control mice | Arthritis model mice | | | Fastigium arthritis model mice (12w) | Balbc, 20w w/o bacteria admin., 3w after bacteria admin. |
| | | | | w/o bacteria admin. 20w | 3w | 12w (fastigium) | | |
| * | G protein-coupled receptor 183 ( Epstein-Barr virus induced gene 2 ) | 1457691-at | 1755.0 | 1427.0 | 1853.2 | 4033.0 | 2.3 | 0.6645 |
| * | B-cell leukemia/ lymphoma 2 related protein A1a, B-cell leukemia/ lymphoma 2 related protein A1b, B-cell leukemia/ lymphoma 2 related protein A1d | 1419004_s_at | 3378.0 | 4243.5 | 4389.7 | 45297.1 | 13.4 | 0.6260 |
| * | Fc receptor, IgG, low affinity IIb | 1435477_s_at | 6927.7 | 14380.3 | 15590.6 | 81184.0 | 11.7 | 0.6223 |

[Table 4-2]

| Gene title | Affymetrix Probe Set ID | Expression level (vs. GAPDH) | | | | Ratio of expression (vs. control) | Correlation coefficient (vs. Il17a) |
|---|---|---|---|---|---|---|---|
| | | Control mice | Arthritis model mice | | | Fastigium arthritis model mice (12w) | Balbc, 20w w/o bacteria admin., 3w after bacteria admin. |
| | | | w/o bacteria admin. 20w | 3w | 12w (fastigium) | | |
| Podoplanin | 1419309_at | 14163.2 | 11263.2 | 14215.8 | 63518.0 | 4.5 | 0.4973 |
| SH3 and PX domains 2B | 1435644_at | 9179.2 | 4845.8 | 9025.3 | 19073.4 | 2.1 | 0.4561 |
| Protein kinase inhibitor beta, cAMP dependent, testis specific | 1421137_a_at | 1208.6 | 1380.7 | 1349.7 | 5608.1 | 4.6 | 0.3641 |
| | 1421138_a_at | 131.5 | 256.0 | 198.2 | 472.7 | 3.6 | 0.0601 |
| Stabilin 1 | 1450199_a_at | 2635.7 | 3637.2 | 3381.6 | 6636.8 | 2.5 | 0.2899 |
| High mobility group AT-hook 2, pseudogene 1 | 1440559_at | 1984.9 | 3068.6 | 2639.5 | 5170.4 | 2.6 | 0.1378 |
| Transforming growth factor beta induced | 1437463_x_at | 28975.2 | 26002.5 | 27863.1 | 75696.1 | 2.6 | 0.1253 |
| | 1448123_s_at | 51505.3 | 45384.4 | 45217.8 | 108869.5 | 2.1 | -0.5359 |
| Disabled homolog 2 | 1423805_at | 3857.4 | 3084.9 | 3452.8 | 8102.0 | 2.1 | -0.0462 |
| Interleukin 27 receptor A | 1449508_at | 73.2 | 313.7 | 155.7 | 1432.4 | 19.6 | -0.1598 |
| Carboxypeptidase D | 1447392_s_at | 80.2 | 170.3 | 110.3 | 207.3 | 2.6 | -0.1704 |
| Chemokine, CC motif, ligand 20 | 1422029_at | 1071.9 | 47.7 | 406.6 | 2163.0 | 2.0 | -0.1887 |
| Immunoglobulin heavy chain (gamma polypeptide) | 1424631_a_at | 620.0 | 1960.4 | 719.3 | 1567.7 | 2.5 | -0.4245 |
| Myosin III B | 1459299_at | 154.1 | 214.7 | 156.6 | 326.2 | 2.1 | -0.4504 |
| Immunoglobulin heavy chain complex, Immunoglobulin heavy chain 1a (serum IgG2a), | 1421653_a_at | 11977.9 | 63907.6 | 12111.0 | 29433.2 | 2.5 | -0.4817 |

(continued)

| Gene title | Affymetrix Probe Set ID | Expression level (vs. GAPDH) | | | | Ratio of expression (vs. control) | Correlation coefficient (vs. Il17a) |
|---|---|---|---|---|---|---|---|
| | | Control mice | Arthritis model mice | | | Fastigium arthritis model mice (12w) | Balbc, 20w w/o bacteria admin., 3w after bacteria admin. |
| | | | w/o bacteria admin. 20w | 3w | 12w (fastigium) | | |
| Immunoglobulin heavy chain 2 (serum IgA), Immunoglobulin heavy chain Ia, Immunoglobulin heavy chain (J558 family), Immunoglobulin heavy chain (gamma polypeptide), similar to immunoglobulin mu-chain, similar to immunoglobulin heavy chain V region3 precursor, Immunoglobulin heavy chain variable region, similar to immunoglobulin heavy chain V region 102 precursor | | | | | | | |
| Killer cell lectin-like receptor subfamily B member 1F | 1457722_at | 296.8 | 540.1 | 292.3 | 1113.9 | 3.8 | -0.4976 |
| Uridine phosphorylase 1 | 1448562_at | 1105.4 | 858.5 | 859.8 | 10087.0 | 9.1 | -0.5120 |
| Immunoglobulin joining chain | 1424305_at | 10933.4 | 48147.6 | 8672.8 | 75764.9 | 6.9 | -0.5277 |
| EH-domain containing 3 | 1417235_at | 2007.9 | 2060.6 | 1997.2 | 4712.8 | 2.3 | -0.6156 |
| Ras-related associated with diabetes | 1422562_at | 552.7 | 496.7 | 476.3 | 4908.7 | 8.9 | -0.7193 |
| Exostoses (multiple) 1 | 1458296_at | 1261.5 | 1234.4 | 646.9 | 5655.4 | 4.5 | -0.9998 |

b) encephalitis model mice

Pearson product-moment correlation coefficients between the expression levels of IL-17A gene and the genes identified as described under point 4.1) above were calculated in the control mice and the encephalitis model mice at 9, 14, 18 and 24 days after the antigen emulsion administration.
Based on the calculated coefficients and the Condition 2, the genes whose expressions correlate with that of IL-17A gene were identified in the encephalitis model mice. The results are shown in Table 5 with asterisks.

3) Identification according to the correlation between pathological conditions and gene expression levels in encephalitis model mice

In encephalitis models, encephalomyelitis inflammation symptoms appear at 10 to 14 days after the administration of the antigen emulsion, and the symptoms are remitted and disappear at 15 to 20 days after the administration. Thus, the present inventors focused on the correlation between the pathological conditions and expression levels of the genes. Namely, genes whose expression increases at the fastigium and decreases at the remission are identified as genes correlating to the pathological conditions in encephalitis model mice (hereinafter referred to as "Condition 3") provided that the ratio of the gene expression level at the remission to that at the fastigium is 0.7 or less.
Among the genes identified in the above point 4. 2)b), the genes which are highly expressed in encephalitis model mice were identified according to the Condition 3. The results are shown in Table 5 with #.

[Table 5-1]

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| # * | Transforming growth factor beta induced | 1448123_s_at | 931.4 | 1295.7 | 25411.1 | 2483.9 | 1726.7 | 27.3 | 0.997 | 9.8 |
| | | 1415871_at | 565.4 | 688.6 | 13625.9 | 1407.2 | 893.6 | 24.1 | 0.997 | 10.3 |
| | | 1456250_x_at | 987.4 | 1481.4 | 21477.8 | 2864.4 | 2021.7 | 21.8 | 0.998 | 13.3 |
| | | 1437463_x_at | 1289.2 | 1725.3 | 11667.4 | 2831.1 | 2230.1 | 9.1 | 0.999 | 24.3 |
| # * | Tumor necrosis factor receptor superfamily, member 14 | 1452425_at | 38.7 | 47.8 | 522.3 | 69.3 | 55.0 | 13.5 | 0.997 | 13.3 |
| # * | Fc receptor, IgG, low affinity IIb | 1435477_s_at | 568.2 | 967.9 | 28718.8 | 4457.2 | 2623.5 | 50.5 | 1.000 | 15.5 |
| # * | apolipoprotein L 7b ( expressed sequence BC085284 ) , apolipoprotein L7e ( similar to apolipoprotein L, 3 ) | 1436271_at 28.4 | 14.9 | 34.8 | 190.6 | 34.8 | 25.7 | 12.8 | 0.995 | 18.3 |
| # * | Tissue inhibitor of metalloproteinase 1 | 1460227_at | 261.2 | 348.4 | 22092.3 | 4481.1 | 2118.4 | 84.6 | 0.998 | 20.3 |
| # | B-cell leukemia/lymphoma 2 related protein A1a, * B-cell leukemia/lymphoma 2 related protein A1b, B-cell leukemia/lymphoma 2 related protein A1d | 1419004_s_at | 820.8 | 921.9 | 16970.5 | 3842.2 | 2216.4 | 20.7 | 0.998 | 22.6 |
| # * | UDP glucuronosyltransferase 1 family, polypeptide A2, UDP glucuronosyltransferase 1 family, polypeptide A6A, | 1426260_a_at | 359.2 | 451.7 | 6567.0 | 1505.3 | 1014.4 | 18.3 | 0.999 | 22.9 |

(continued)

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| | UDP glucuronosyltransferase 1 family, polypeptide A6B, | | | | | | | | | |
| | UDP glucuronosyltransferase 1 family, polypeptide A10, UDP glucuronosyltransferase 1 family, polypeptide A7C, UDP glucuronosyltransferase 1 family, polypeptide A5, | 1426261_s_at | 149.4 | 217.6 | 1963.8 | 502.1 | 289.9 | 13.1 | 0.997 | 25.6 |
| | UDP glucuronosyltransferase 1 family, polypeptide A9, UDP glucuronosyltransferase 1 family, polypeptide A1, similar to UDP glycosyltransferase 1 family, polypeptide A8 | 1424783_a_at | 497.4 | 572.1 | 3717.2 | 1253.5 | 926.5 | 7.5 | 0.995 | 33.7 |
| # * | Interleukin 17A | 1421672_at | 83.9 | 80.0 | 655.9 | 155.4 | 129.5 | 7.8 | 1.000 | 23.7 |
| # * | Acid phosphatase, prostate | 1441975_at | 157.3 | 111.4 | 510.4 | 149.1 | 114.7 | 3.2 | 0.987 | 29.2 |
| | | 1453943_a_at | 53.5 | 49.8 | 185.2 | 59.0 | 56.4 | 3.5 | 0.997 | 31.8 |
| # * | Uridine phosphorylase 1 | 1448562_at | 535.6 | 580.5 | 2198.0 | 668.5 | 544.2 | 4.1 | 0.995 | 30.4 |
| | | 1425603_at | 1254.4 | 1852.5 | 9356.9 | 3350.5 | 2559.2 | 7.5 | 0.992 | 35.8 |
| # * | Transmembrane protein 176A | 1423909_at | 5751.3 | 7428.5 | 21118.2 | 12568.0 | 10429.2 | 3.7 | 0.946 | 59.5 |
| | | 1441811_x_at | 2605.8 | 3076.9 | 9358.1 | 5803.4 | 4888.6 | 3.6 | 0.929 | 62.0 |
| # * | UDP-GlcNAc:betaGal | 1425128_at | 141.1 | 72.6 | 409.1 | 149.6 | 171.7 | 2.9 | 0.974 | 36.6 |

(continued)

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| | beta-1,3-N-acetylglucosaminyltransferase 8 | | | | | | | | | |
| # * | Interleukin 1 receptor 1 | 1448950_at | 192.8 | 390.6 | 923.4 | 339.4 | 276.5 | 4.8 | 0.964 | 36.8 |
| # * | Cannabinoid receptor 2 | 1450476_at | 42.8 | 99.8 | 545.1 | 219.9 | 166.5 | 12.7 | 0.974 | 40.3 |
| | | 1420498_a_at | 1210.3 | 1597.6 | 5167.7 | 2100.7 | 1856.7 | 4.3 | 0.994 | 40.7 |
| # * | Disabled homolog 2 | 1430604_a_at | 348.4 | 336.5 | 954.6 | 391.6 | 322.5 | 2.7 | 0.994 | 41.0 |
| | | 1423805_at | 461.2 | 500.5 | 960.0 | 569.7 | 428.9 | 2.1 | 0.976 | 59.3 |

[Table 5-2]

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| # * | G protein-coupled receptor 15 | 1431296_at | 45.7 | 53.9 | 194.7 | 81.8 | 47.6 | (14d) 4.3 | 0.987 | 42.0 |
| # * | Interleukin 22, Interleukin tifb | 1427624_s_at | 19.3 | 25.4 | 71.1 | 30.7 | 14.4 | 3.7 | 0.963 | 43.2 |
| # * | Interleukin 27 receptor A | 1449508_at | 103.3 | 57.2 | 432.6 | 198.9 | 142.1 | 4.2 | 0.971 | 46.0 |
| # * | RIKEN CDNA 6030439006 gene | 1443078_at | 26.3 | 28.9 | 59.2 | 28.2 | 36.1 | 2.3 | 0.967 | 47.7 |
| # * | Cytochrome P450, family 1, subfamily b, polypeptide 1 | 1416612_at | 435.9 | 506.8 | 1415.6 | 683.5 | 529.4 | 3.2 | 0.992 | 48.3 |
| # * | Phosphatase, orphan 1 ( expressed sequence AI447357, ABI gene family, member 3 ) | 1457063_at | 164.6 | 194.8 | 487.0 | 239.2 | 174.8 | 3.0 | 0.987 | 49.1 |

(continued)

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| # * | Solute carrier family 38, member 6 ( expressed sequence AW322671 ) | 1457266_at | 784.7 | 914.8 | 2305.4 | 1143.5 | 1020.0 | 2.9 | 0.994 | 49.6 |
| # * | Cysteine-rich secretory protein LCCL domain containing 2 | 1460458_at | 442.3 | 513.3 | 1157.8 | 574.6 | 456.4 | 2.6 | 0.989 | 49.6 |
| | | 1434758_at | 442.3 | 513.3 | 1970.7 | 983.1 | 456.4 | 2.6 | 0.989 | 49.9 |
| | | 1437056_x_at | 1505.7 | 2007.0 | 5533.9 | 3267.5 | 2847.9 | 3.7 | 0.944 | 59.0 |
| * | Glucosaminyl (N-acetyl) transferase 2, I-branching enzyme | 1425503_at | 1036.0 | 1308.3 | 2675.7 | 1353.5 | 1254.7 | 2.6 | 0.988 | 50.6 |
| # | | 1451733_at | 135.9 | 132.8 | 399.5 | 211.7 | 158.1 | 2.9 | 0.986 | 53.0 |
| | | 1421415_s_at | 463.2 | 469.5 | 921.0 | 534.0 | 462.9 | 2.0 | 0.995 | 58.0 |
| # * | Ras-related associated with diabetes | 1422562_at | 95.3 | 113.9 | 415.9 | 212.1 | 158.7 | 4.4 | 0.973 | 51.0 |
| # * | Stabilin 1 | 1450199_a_at | 275.5 | 335.4 | 902.7 | 498.7 | 342.5 | 3.3 | 0.974 | 55.3 |
| # * | Matrix metallopeptidase 13 | 1417256_at | 33.6 | 39.3 | 69.4 | 38.7 | 39.3 | 2.1 | 0.987 | 55.7 |
| # * | Bone morphogenetic protein 1 | 1427457_a_at | 214.6 | 297.6 | 721.7 | 430.3 | 363.1 | 3.4 | 0.952 | 59.6 |
| | | 1426238_at | 819.5 | 1006.6 | 1936.5 | 1478.6 | 1083.1 | 2.4 | 0.897 | 76.4 |

(continued)

| | Gene title | Affymetrix Probe Set ID | Expression level (vs. GAPDH) | | | | | Ratio of expression (vs. control) | Correlation coefficient (vs. Il17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| # * | Transcription factor 7 (T-cell specific) | 1450461_at | 106.2 | 59.2 | 220.1 | 132.9 | 221.6 | 2.1 | 0.625 | 60.4 |
| | | 1433471_at | 360.3 | 485.4 | 910.9 | 562.6 | 492.6 | 2.5 | 0.963 | 61.8 |
| # * | Transmembrane protein 176B | 1418004_a_at | 12557.8 | 14813.1 | 35782.1 | 21865.2 | 17937.1 | 2.8 | 0.964 | 61.1 |
| # * | Carbonic anhydrase 13 | 1421307_at | 711.8 | 868.8 | 1637.3 | 1034.2 | 911.2 | 2.3 | 0.974 | 63.2 |
| # * | Lymphocyte antigen 6 complex, locus K | 1452855_at | 194.4 | 212.9 | 597.3 | 389.6 | 304.2 | 3.1 | 0.933 | 65.2 |
| # * | SH3 and PX domains 2B | 1435644_at | 1011.1 | 1070.4 | 2010.2 | 1320.1 | 1129.0 | 2.0 | 0.985 | 65.7 |
| # * | WW domain containing transcription regulator 1 | 1437155_a_at | 1105.7 | 1405.3 | 2476.6 | 1684.1 | 1612.0 | 2.2 | 0.939 | 68.0 |

[Table 5-3]

| | Gene title | Affymetrix Probe Set ID | Expression level (vs. GAPDH) | | | | | Ratio of expression (vs. control) | Correlation coefficient (vs. ‖17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| * | RIKEN cDNA 1300007F04 gene | 1453474_at | 202.0 | 232.2 | 476.7 | 345.7 | 204.4 | 2.4 | 0.907 | 72.5 |
| * | Podoplanin | 1419309_at | 1821.5 | 1887.5 | 5479.8 | 4045.8 | 3259.8 | 3.0 | 0.864 | 73.8 |
| * | Patatin-like phospholipase domain containing 7 | 1451361_a_at | 1355.5 | 1425.8 | 2895.4 | 2151.7 | 1878.3 | 2.1 | 0.912 | 74.3 |
| * | Retinol binding protein 1, cellular, similar to cellular retinol binding protein I | 1448754_at | 1225.1 | 1088.4 | 4167.0 | 4012.7 | 2750.5 | 3.4 | 0.678 | 96.3 |
| | Bactericidal/ permeability-increasing protein-like 2 | 1437232_at | 23.9 | 60.3 | 62.8 | 66.4 | 37.2 | 2.6 | 0.426 | 105.8 |
| | Immunoglobulin heavy chain complex, Immunoglobulin heavy chain 1a (serum IgG2a), Immunoglobulin heavy chain 2 (serum IgA), | | | | | | | | | |

(continued)

| | Gene title | Affymetrix Probe Set ID | Expression level (vs. GAPDH) | | | | | Ratio of expression (vs. control) | Correlation coefficient (vs. ‖17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| | Immunoglobulin heavy chain Ia, Immunoglobulin heavy chain (J558 family), Immunoglobulin heavy chain (gamma polypeptide), similar to immunoglobulin mu-chain, similar to immunoglobulin heavy chain V region3 precursor, Immunoglobulin heavy chain variable region, similar to immunoglobulin heavy chain V region 102 precursor | 1421653_a_at | 357.7 | 351.8 | 877.2 | 1093.9 | 901.1 | 2.5 | 0.386 | 124.7 |
| | RI KEN cDNA 2310002J15 gene | 1450532_at | 19.5 | 55.9 | 39.8 | 83.6 | 37.2 | 2.0 | -0.090 | 210.1 |

(continued)

| | Gene title | Affymetrix Probe Set ID | Expression level (vs. GAPDH) | | | | | Ratio of expression (vs. control) | Correlation coefficient (vs. ‖17a) | Ratio of expression (remission vs. fastigium) |
|---|---|---|---|---|---|---|---|---|---|---|
| | | | Control mice | Encephalitis model mice | | | | Fastigium encephalitis model mice (14d) | Control, 9d, 14d, 18d, 24d | 14d, 18d |
| | | | | 9d | 14d | 18d | 24d | | | |
| | Immunoglobulin heavy chain 3, Immunoglobulin heavy chain (gamma polypeptide) | 1426174_s_at | 44.1 | 77.4 | 154.2 | 369.7 | 173.0 | 3.5 | 0.081 | 239.7 |
| | Immunoglobulin heavy chain (gamma polypeptide) | 1424631_a_at | 49.7 | 46.7 | 107.5 | 531.8 | 240.5 | 2.2 | -0.117 | 494.5 |

4) Summary

Gene titles (i.e. functional annotations) of the genes identified according to the Conditions 2 and 3 are shown in Table 6, which are highly expressed in arthritis and encephalitis model mice. In the table, a circle (˚) corresponds to the gene satisfying the Condition in the indicated model mice and "-" corresponds to the gene that does not satisfy the Condition. The genes are marked with asterisks when they satisfy the Conditions in both arthritis and encephalitis model mice.

[Table 6]

| | Gene title | Arthritis | Encepha litis |
|---|---|---|---|
| | RIKEN cDNA 6030439D06 gene | - | ○ |
| | RIKEN cDNA 9030418K01 gene | ○ | - |
| | Acid phosphatase, prostate | - | ○ |
| * | Apolipoprotein L7b ( expressed sequence BC085284 )<br>Apolipoprotein L7e ( similar to apolipoprotein L, 3 ) | ○ | ○ |
| | UDP-GlcNAc:betaGal beta-1,3-N-acetylglucosaminyltransferase 8 | - | ○ |
| * | B-cell leukemia/lymphoma 2 related protein A1a,<br>B-cell leukemia/lymphoma 2 related protein A1b,<br>B-cell leukemia/lymphoma 2 related protein A1d | ○ | ○ |
| | Bone morphogenetic protein 1 | - | ○ |
| | C1q and tumor necrosis factor related protein 3 | ○ | - |
| | Carbonic anhydrase 13 | - | ○ |
| * | Cannabinoid receptor 2 | ○ | ○ |
| * | Cysteine-rich secretory protein LCCL domain containing 2 | ○ | ○ |
| | Cytochrome P450, family 1, subfamily b, polypeptide 1 | - | ○ |
| | Disabled homolog 2 | - | ○ |
| * | Fc receptor, IgG, low affinity IIb | ○ | ○ |
| | Glucosaminyl (N-acetyl) transferase 2, I-branching enzyme | - | ○ |
| | G protein-coupled receptor 15 | - | ○ |
| | G protein-coupled receptor 183 ( Epstein-Barr virus induced gene 2 ) | ○ | - |
| * | Interleukin 17A | ○ | ○ |
| * | Interleukin 1 receptor 1 | ○ | ○ |
| | Interleukin 22, Interleukin tifb | - | ○ |
| | Interleukin 27 receptor A | - | ○ |
| | Kinesin family member 5C | ○ | - |
| | Retinol binding protein 1, cellular,<br>similar to cellular retinol binding protein I | ○ | - |
| | UDP glucuronosyltransferase 1 family, polypeptide A2,<br>UDP glucuronosyltransferase 1 family, polypeptide A6A,<br>UDP glucuronosyltransferase 1 family, polypeptide A6B,<br>UDP glucuronosyltransferase 1 family, polypeptide A10,<br>UDP glucuronosyltransferase 1 family, polypeptide A7C,<br>UDP glucuronosyltransferase 1 family, polypeptide A5,<br>UDP glucuronosyltransferase 1 family, polypeptide A9, | - | ○ |

(continued)

| | Gene title | Arthritis | Encepha litis |
|---|---|---|---|
| | UDP glucuronosyltransferase 1 family, polypeptide A1, similar to UDP glycosyltransferase 1 family, polypeptide A8 | | |
| | Lumican | ○ | - |
| | Lymphocyte antigen 6 complex, locus K | - | ○ |
| * | Matrix metallopeptidase 13 | ○ | ○ |
| | Phosphodiesterase 5A (cGMP-specific) | ○ | - |
| | Phosphatase, orphan 1( expressed sequence AI447357, ABI gene family, member 3 ) | - | ○ |
| | Ras-related associated with diabetes | - | ○ |
| | Serine (or cysteine) peptidase inhibitor, clade B, member 1a | ○ | - |
| | SH3 and PX domains 2B | - | ○ |
| * | Solute carrier family 38, member 6 ( expressed sequence AW322671 ) | ○ | ○ |
| | Stabilin 1 | - | ○ |
| | Synaptotagmin XI | ○ | - |
| | Transcription factor 7 (T-cell specific) | - | ○ |
| | Transforming growth factor beta induced | - | ○ |
| * | Tissue inhibitor of metalloproteinase 1 | ○ | ○ |
| * | Transmembrane protein 176A | ○ | ○ |
| | Transmembrane protein 176B | - | ○ |
| | Tumor necrosis factor receptor superfamily, member 14 | - | ○ |
| | Uridine phosphorylase 1 | - | ○ |
| | WW domain containing transcription regulator 1 | - | ○ |

The present application relates to Japanese Patent Application No. 2008-048197 filed on February 28, 2008, whose claims, specification and abstract are incorporated herein by reference.

## Claims

1. A polynucleotide marker for detecting Th 17 cells which is a polynucleotide selected from the group consisting of:

a gene encoding a cytokine selected from the group consisting of Interleukin 17A; Interleukin 22; and Interleukin tifb;
a gene encoding a chemokine which is Chemokine, CC motif, ligand 20;
a gene encoding a membrane protein selected from the group consisting of Interleukin 17 receptor E; Interleukin 1 receptor 1; Interleukin 27receptor A; G protein-coupled receptor 15; Stabilin 1; Podoplanin; Transmembrane and immunoglobulin domain containing 1; Melanocortin 2 receptor; Transmembrane protein 176A; Progestin and adipoQ receptor family member VIII; Claudin domain containing 1; ELOVL family member 7; Lymphocyte antigen 6 complex, locus K; G protein-coupled receptor 183 (Epstein-Barr virus induced gene 2); Killer cell lectin-like receptor subfamily B member 1F; Transferrin receptor 2; Neuron specific gene family member 2; Transmembrane protein 176B; Amyloid beta (A4) precursor-like protein 2; Immunoglobulin joining chain; Adhesion molecule with Ig like domain 2; Fc receptor, IgG, low affinity IIb; Cannabinoid receptor 2; Tumor necrosis factor receptor superfamily, member 14; Aquaporin 3; C1q and tumor necrosis factor related protein 3; Synaptotagmin XI; Potassium channel tetramerisation domain containing 12; Apolipoprotein L 7b (expressed sequence BC085284); Apolipoprotein L7e (similar to apolipoprotein L, 3); Solute carrier family 34 (member 3); Retinol binding protein 1, cellular; similar to cellular retinol binding protein I; Potassium large conductance calcium-activated channel (subfamily M, beta member 4); similar to calcium activated potassium channel beta 4 subunit; SYS 1 Golgi-localized integral membrane protein homolog (RIKEN cDNA 2610042014 gene); and Solute carrier family 38, member 6 (expressed sequence AW322671);
a gene encoding a transcription/translation factor selected from the group consisting of POU domain, class 2, associating factor 1; Transcription factor 7 (T-cell specific); WW domain containing transcription regulator 1; Trichorhinophalangeal syndrome I; Centrosomal protein 290; and Ataxin 2 binding protein 1;
a gene encoding a signaling molecule selected from the group consisting of Ras-related associated with diabetes; Breast cancer anti-estrogen resistance 3; Rab38 (member of RAS oncogene family); Centaurin, gamma 2; SH3 and PX domains 2B; FERM, RhoGEF and pleckstrin domain protein 2; Disabled homolog 2; B-cell leukemia/ lymphoma 2 related protein A1a; B-cell leukemia/ lymphoma 2 related protein A1b; and B-cell leukemia/ lymphoma 2 related protein A1d;
a gene encoding an adhesion molecule which is Transforming growth factor beta induced;
a gene encoding an enzyme selected from the group consisting of Cytochrome P450, family 1, subfamily b, polypeptide 1; EH-domain containing 3; Matrix metallopeptidase 13; Carboxypeptidase D; Carbonic anhydrase 13; Glucosaminyl (N-acetyl) transferase 2,
1-branching enzyme; UDP glucuronosyltransferase 1 family, polypeptide A2; UDP glucuronosyltransferase 1 family, polypeptide A6A; UDP glucuronosyltransferase 1 family, polypeptide A6B; UDP glucuronosyltransferase 1 family, polypeptide A10; UDP glucuronosyltransferase 1 family, polypeptide A7C; UDP glucuronosyltransferase 1 family, polypeptide A5; UDP glucuronosyltransferase 1 family, polypeptide A9; UDP glucuronosyltransferase 1 family, polypeptide A1; Similar to UDP glycosyltransferase 1 family, polypeptide A8; UDP-GlcNAc: betaGal beta-1,3-N-acetylglucosaminyltransferase 8; Bone morphogenetic protein 1; Uridine phosphorylase 1; Myosin III B; beta-site APP-cleaving enzyme 2; Mast cell protease 1; COX10 homolog, cytochrome c oxidase assembly protein, heme A: farnesyltransferase; Dynamin 3; Acid phosphatase, prostate; Phosphodiesterase 5A (cGMP-specific); Patatin-like phospholipase domain containing 7; RIKEN cDNA 1300007F04 gene; RIKEN cDNA 1810062018 gene; Phosphatase, orphan 1 (expressed sequence AI447357, ABI gene family, member 3); and Exostoses (multiple) 1;
a gene encoding an enzyme inhibitor selected from the group consisting of Serine (or cysteine) peptidase inhibitor, clade B, member 1a; Protein phosphatase 1, regulatory (inhibitor) subunit 14c; Protein kinase inhibitor beta (cAMP dependent, testis specific); Tissue inhibitor of metalloproteinase 1; Serine (or cysteine) peptidase inhibitor, clade I, member 1; Amyloid beta (A4) precursor protein; and WAP four-disulfide core domain 2;
a gene encoding a secretory protein which is Cysteine-rich secretory protein LCCL domain containing 2;
a gene encoding a structural protein selected from the group consisting of Plastin 1 (expressed sequence AI427122); immunoglobulin heavy chain complex; immunoglobulin heavy chain 1a (serum IgG2a); immunoglobulin heavy chain 2 (serum IgA); immunoglobulin heavy chain Ia; immunoglobulin heavy chain (J558 family); immunoglobulin heavy chain (gamma polypeptide); similar to immunoglobulin mu-chain; similar to immunoglobulin heavy chain V region 3 precursor; immunoglobulin heavy chain variable region; similar to immunoglobulin heavy chain V region 102 precursor; immunoglobulin heavy chain 3; Nebulette; Lumican; Bactericidal/permeability-increasing protein-like 2; Kelch-like 8; Tripartite motif protein 2; PDZ and LIM domain 5; Keratin 86; Kinesin family member 3C; Kinesin family member 1B; and Kinesin family member 5C;

a gene selected from the group consisting of Sex comb on midleg-like 4; High mobility group AT-hook 2, pseudogene 1; RIKEN cDNA 2310007L24 gene; RIKEN cDNA 2310002J 15 gene; Family with sequence similarity 101, member B (RIKEN cDNA 1500005K14 gene); expressed sequence AI646023; and GRAM domain containing 3; and

an expressed sequence tag (EST) selected from TOX high mobility group box family member 2 (expressed sequence AI851523); RIKEN cDNA 6030439D06 gene; RIKEN cDNA 9030418K01 gene; expressed sequence AU015680; a polynucleotide having the sequence of SEQ ID NO: 1; and a polynucleotide having the sequence of SEQ ID NO: 2.

**2.** The polynucleotide marker for detecting Th 17 cells according to claim 1, which is selected from the group consisting of:

a gene encoding a cytokine selected from the group consisting of Interleukin 17A; Interleukin 22; and Interleukin tifb;

a gene encoding a membrane protein selected from the group consisting of Interleukin 1 receptor 1; Interleukin 27receptor A; G protein-coupled receptor 15; Stabilin 1; Apolipoprotein L 7b (expressed sequence BC085284); Apolipoprotein L7e (similar to apolipoprotein L, 3); C1q and tumor necrosis factor related protein 3; Cannabinoid receptor 2; Fc receptor, IgG, low affinity IIb; G protein-coupled receptor 183 (Epstein-Barr virus induced gene 2); Retinol binding protein 1, cellular; similar to cellular retinol binding protein I; Lymphocyte antigen 6 complex, locus K; Solute carrier family 38, member 6 (expressed sequence AW322671); Synaptotagmin XI; Transmembrane protein 176A; Transmembrane protein 176B; and Tumor necrosis factor receptor superfamily, member 14;

a gene encoding a transcription/translation factor selected from Transcription factor 7 (T-cell specific); and WW domain containing transcription regulator 1;

a gene encoding a signaling molecule selected from the group consisting of B-cell leukemia/lymphoma 2 related protein A1a; B-cell leukemia/lymphoma 2 related protein A1b; B-cell leukemia/ lymphoma 2 related protein A1d; Disabled homolog 2; Ras-related associated with diabetes; and SH3 and PX domains 2B;

a gene encoding an adhesion molecule which is Transforming growth factor beta induced;

a gene encoding an enzyme selected from the group consisting of Acid phosphatase, prostate; UDP-GlcNAc: betaGal beta-1,3-N-acetylglucosaminyltransferase 8; Bone morphogenetic protein 1; Carbonic anhydrase 13; Cytochrome P450, family 1, subfamily b, polypeptide 1; Glucosaminyl (N-acetyl) transferase 2, 1-branching enzyme; UDP glucuronosyltransferase 1 family, polypeptide A2; UDP glucuronosyltransferase 1 family, polypeptide A6A; UDP glucuronosyltransferase 1 family, polypeptide A6B; UDP glucuronosyltransferase 1 family, polypeptide A10; UDP glucuronosyltransferase 1 family, polypeptide A7C; UDP glucuronosyltransferase 1 family, polypeptide A5; UDP glucuronosyltransferase 1 family, polypeptide A9; UDP glucuronosyltransferase 1 family, polypeptide A1; Similar to UDP glycosyltransferase 1 family, polypeptide A8; Matrix metallopeptidase 13; Phosphodiesterase 5A (cGMP-specific); Phosphatase, orphan 1 (expressed sequence AI447357, ABI gene family, member 3); and Uridine phosphorylase 1;

a gene encoding an enzyme inhibitor selected from Serine (or cysteine) peptidase inhibitor, clade B, member 1a; and Tissue inhibitor of metalloproteinase 1;

a gene encoding a secretory protein which is Cysteine-rich secretory protein LCCL domain containing 2;

a gene encoding a structural protein selected from Kinesin family member 5C; and Lumican; and

an expressed sequence tag (EST) selected from RIKEN cDNA 6030439D06 gene; and RIKEN cDNA 9030418K01 gene.

**3.** The polynucleotide marker for detecting Th17 cells according to claim 1, which is selected from the group consisting of:

a gene encoding a cytokine which is Interleukin 17A;

a gene encoding a membrane protein selected from the group consisting of Interleukin 1 receptor 1; Apolipoprotein L 7b (expressed sequence BC085284); Apolipoprotein L7e (similar to apolipoprotein L, 3); Cannabinoid receptor 2; Fc receptor, IgG, low affinity IIb; Solute carrier family 38, member 6 (expressed sequence AW322671); and Transmembrane protein 176A;

a gene encoding a signaling molecule selected from the group consisting of B-cell leukemia/lymphoma 2 related protein A1a; B-cell leukemia/lymphoma 2 related protein A1b; and B-cell leukemia/ lymphoma 2 related protein A1d;

a gene encoding an enzyme which is Matrix metallopeptidase 13;

a gene encoding an enzyme inhibitor which is Tissue inhibitor of metalloproteinase 1; and

a gene encoding a secretory protein which is Cysteine-rich secretory protein LCCL domain containing 2.

4. A protein marker for detecting Th17 cells consisting of a protein encoded by the gene defined in claim 1.

5. A method for detecting Th17 cells **characterized in that** it comprises detecting the presence of the polynucleotide marker for detecting Th 17 cells according to claim 1 or the protein marker for detecting Th17 cells according to claim 4 in a sample containing cells.

6. A DNA chip or microarray **characterized in that** it comprises a probe which specifically hybridizes to the polynucleotide marker for detecting Th 17 cells according to claim 1.

7. A nucleic acid primer for amplifying the polynucleotide marker for detecting Th 17 cells according to claim 1.

8. An antibody **characterized in that** it specifically binds to the protein marker for detecting Th17 cells according to claim 4.

9. A kit for detecting Th17 cells **characterized in that** it comprises the DNA chip or microarray according to claim 6, the nucleic acid primer according to claim 7 or the antibody according to claim 8.

**INTERNATIONAL SEARCH REPORT**

International application No.
PCT/JP2009/053700

A. CLASSIFICATION OF SUBJECT MATTER
*C12Q1/68*(2006.01)i, *C12N15/09*(2006.01)i, *C12Q1/04*(2006.01)i, *G01N33/48* (2006.01)i, *G01N33/53*(2006.01)i

According to International Patent Classification (IPC) or to both national classification and IPC

B. FIELDS SEARCHED

Minimum documentation searched (classification system followed by classification symbols)
C12Q1/68, C12N15/09, C12Q1/04, G01N33/48, G01N33/53

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | Francesco ANNUNZIATO et al., Phenotypic and functional features of human Th17 cells, The Journal of Experimental Medicine, 2007, vol.204, No.8, p.1849-1861, full text | 1-9 |
| A | LI Jiawen et al., Expression of Th17 Cytokines in Skin Lesions of Patients with Psoriasis, Journal of Huazhong University of Science and Technology, 2007, 27(3), p.330-332 | 1-9 |
| A | Richard H. DUERR et al., A Genome-Wide Association Study Identifies IL23R as an Inflammatory Bowel Disease Gene, SCIENCE, 2006, Vol.314, p.1461-1463 | 1-9 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 March, 2009 (31.03.09) | 07 April, 2009 (07.04.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/053700

C (Continuation). DOCUMENTS CONSIDERED TO BE RELEVANT

| Category* | Citation of document, with indication, where appropriate, of the relevant passages | Relevant to claim No. |
|---|---|---|
| A | Keiji HIROTA et al., Preferential recruitment of CCR6-expressing Th17 cells to inflamed joints via CCL20 in rheumatoid arthritisand its animal model, The Journal of Experimental Medicine, 2007, Vol.204, No.12, p.2803-2812 | 1-9 |
| A | Yan ZHENG et al., Interleukin-22, a TH17 cytokine, mediates IL-23-induced dermal inflammation and acanthosis, Nature, 2007, Vol.445, p.648-651 | 1-9 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000186046 A
- JP 2007506100 A
- JP 2008048197 A


### Non-patent literature cited in the description


- **Ivanov et al.** *Cell,* 2006, vol. 126, 1121-1133
- **Stumhofer et al.** *Nature Immunology,* 2006, vol. 7, 937-945
- **Wilson et al.** *Nature Immunology,* 2007, vol. 8, 950-957
- **Ivanov et al.** The Orphan Nuclear Receptor RORγt Directs the Differentiation Program of Proinflammatory IL-17+ T Helper Cells. *Cell,* 2006, vol. 126, 1121-1133
- **Stumhofer et al.** Interleukin 27 negatively regulates the development of interleukin 17-producing T helper cells during chronic inflammation of the central nervous system. *Nature Immunology,* 2006, vol. 7, 937-945
- **Wilson et al.** Development, cytokine profile and function of human interleukin 17-producing helper T cells. *Nature Immunology,* 2007, vol. 8, 950-957
- *Nature,* 2003, vol. 426, 454-460

SEQUENCE LISTING

<110> Sysmex Corporation

<120> Marker for detection of IL-17-producing helper T-cell, and method for detection of IL-17-producing helper T-cell

<130> SYSM-042-EP

<150> JP 2008-48197
<151> 2008-02-28

<160> 2

<170> PatentIn version 3.1

<210> 1
<211> 136
<212> DNA
<213> Mus musculus

<220>
<221> misc_feature
<222> (48)..(48)
<223> n=a, c, g or t

<220>
<221> misc_feature
<222> (50)..(50)
<223> n=a, c, g or t

<220>
<221> misc_feature
<222> (74)..(74)
<223> n=a, c, g or t

<220>
<221> misc_feature
<222> (100)..(100)
<223> n=a, c, g or t

<220>
<221> misc_feature
<222> (102)..(102)
<223> n=a, c, g or t

<400> 1
accccacatt ataaaccatt tccagtgatc cttctgtaag aatgtgantn tgcagaggca 60

cacacccacc tgcntcgggt ttttgtggat tcagacctgn tnagggtact tgtgaagtca 120

ccacgttttc agtttt 136

<210> 2
<211> 239
<212> DNA
<213> Mus musculus

<400> 2
ctcactttct acttctaata ctgaatgctg ttttgtttgg gcaggaagac gttcacagac 60

tgttcctttg aattcattgt ctgacctgcc tattggagac aaacctctgc tactcataga 120

attattaata ttttttttcct gtcaatttca gttgacagaa gggataaaga ctcaacttgc 180

actcgcgtat ctgtagtcat atctgccttc aggcatgtgc ccctgtgacc gtttctctg 239